# EUROPEAN PATENT APPLICATION

(11) **EP 3 348 651 A1**
(43) Date of publication of application: **18.07.2018**
(21) Application number: 17151531.5
(22) Date of filing: 13.01.2017
(51) Int. Cl.: C12Q 1/68

(54) **RAPID ANTIMICROBIAL SUSCEPTIBILITY TESTING AND PHYLOGENETIC IDENTIFICATION**

(71) Applicant: Helmholtz-Zentrum für Infektionsforschung GmbH, 38124 Braunschweig (DE)
(72) Inventor: Häußler, Susanne, 38106 Braunschweig (DE); Bruchmann, Sebastian, 38104 Braunschweig (DE); Chesnel, Delphine, 30419 Hannover (DE)
(74) Representative: Fuchs Patentanwälte Partnerschaft mbB

(57) **Abstract**

The present invention relates to a method for determining in a bacterial sample information on both the phylogenetic origin and the antibiotic resistances of bacterial strains. The present invention further relates a kit for performing the method of the present invention.

SEQUENCE LISTING

<110>  Helmholtz-Zentrum für Infektionsforschung GmbH

<120>  Rapid Antimicrobial susceptibility testing and Phylogenetic
       Identification

<130>  AN0003P-EP

<160>  94

<170>  PatentIn version 3.5

<210>  1
<211>  30
<212>  DNA
<213>  Artificial Sequence

<220>
<223>  Primer Sequence

<400>  1
acgttggatg ctattgtgtt ggccgtctac                                        30


<210>  2
<211>  30
<212>  DNA
<213>  Artificial Sequence

<220>
<223>  Primer Sequence

<400>  2
acgttggatg aatccctcga gcgcgagtct                                        30


<210>  3
<211>  19
<212>  DNA
<213>  Artificial Sequence

<220>
<223>  Primer Sequence

<400>  3
gcctaacaag gatgacaag                                                    19


<210>  4
<211>  30
<212>  DNA
<213>  Artificial Sequence

<220>
<223>  Primer Sequence

<400>  4
acgttggatg ccaatattat gcacccggtc                                        30


<210>  5
<211>  30

<212>  DNA
<213>  Artificial Sequence

<220>
<223>  Primer Sequence

<400>  5
acgttggatg gagctggcgg aaaaccagat                                        30


<210>  6
<211>  19
<212>  DNA
<213>  Artificial Sequence

<220>
<223>  Primer Sequence

<400>  6
gggactgagc gggtgcatg                                                    19


<210>  7
<211>  30
<212>  DNA
<213>  Artificial Sequence

<220>
<223>  Primer Sequence

<400>  7
acgttggatg atgttggtgt tcgtttccgc                                        30


<210>  8
<211>  30
<212>  DNA
<213>  Artificial Sequence

<220>
<223>  Primer Sequence

<400>  8
acgttggatg gtttaaaaga cgagcacgga                                        30


<210>  9
<211>  17
<212>  DNA
<213>  Artificial Sequence

<220>
<223>  Primer Sequence

<400>  9
ctgtaatggc gtctgcg                                                      17


<210>  10
<211>  29
<212>  DNA
<213>  Artificial Sequence

<220>
<223>  Primer Sequence

<400>  10
acgttggatg gagaataagc agcaaggat                                         29


<210>  11
<211>  30
<212>  DNA
<213>  Artificial Sequence

<220>
<223>  Primer Sequence

<400>  11
acgttggatg ccatcccact taaagacttg                                        30


<210>  12
<211>  22
<212>  DNA
<213>  Artificial Sequence

<220>
<223>  Primer Sequence

<400>  12
gggcgggtaa aaatgcttgg tt                                                22


<210>  13
<211>  30
<212>  DNA
<213>  Artificial Sequence

<220>
<223>  Primer Sequence

<400>  13
acgttggatg gtttcgtctg gatcgcactg                                        30


<210>  14
<211>  29
<212>  DNA
<213>  Artificial Sequence

<220>
<223>  Primer Sequence

<400>  14
acgttggatg tgggtttcgc ccagcgcat                                         29


<210>  15
<211>  17
<212>  DNA
<213>  Artificial Sequence

<220>
<223>  Primer Sequence

<400>  15
ttagagacac caccacg                                                      17


<210>  16
<211>  30
<212>  DNA
<213>  Artificial Sequence

<220>
<223>  Primer Sequence

<400>  16
acgttggatg gcgctacagt acagcgataa                                        30


<210>  17
<211>  29
<212>  DNA
<213>  Artificial Sequence

<220>
<223>  Primer Sequence

<400>  17
acgttggatg agacggaacg tttcgtctc                                         29


<210>  18
<211>  15
<212>  DNA
<213>  Artificial Sequence

<220>
<223>  Primer Sequence

<400>  18
tcgggcgaac gcggt                                                        15


<210>  19
<211>  30
<212>  DNA
<213>  Artificial Sequence

<220>
<223>  Primer Sequence

<400>  19
acgttggatg aatgctgaat ggagagccga                                        30


<210>  20
<211>  30
<212>  DNA
<213>  Artificial Sequence

<220>
<223>  Primer Sequence

<400>  20
acgttggatg ttgaacagca actcaaccag                                        30

<210>  21
<211>  16
<212>  DNA
<213>  Artificial Sequence

<220>
<223>  Primer Sequence

<400>  21
cggaagcggg gacgga                                                        16


<210>  22
<211>  30
<212>  DNA
<213>  Artificial Sequence

<220>
<223>  Primer Sequence

<400>  22
acgttggatg gatccgatgc tacgagaaag                                         30


<210>  23
<211>  30
<212>  DNA
<213>  Artificial Sequence

<220>
<223>  Primer Sequence

<400>  23
acgttggatg gtttgaacca tgtacacggc                                         30


<210>  24
<211>  15
<212>  DNA
<213>  Artificial Sequence

<220>
<223>  Primer Sequence

<400>  24
acacggctgg accat                                                         15


<210>  25
<211>  30
<212>  DNA
<213>  Artificial Sequence

<220>
<223>  Primer Sequence

<400>  25
acgttggatg tggcccgtcc aaagacaaca                                         30


<210>  26
<211>  30

<212>  DNA
<213>  Artificial Sequence

<220>
<223>  Primer Sequence

<400>  26
acgttggatg atcaggcctt caacgatacc                                        30


<210>  27
<211>  22
<212>  DNA
<213>  Artificial Sequence

<220>
<223>  Primer Sequence

<400>  27
gaggggaaac gatgtcctgg cc                                                22


<210>  28
<211>  996
<212>  DNA
<213>  Klebsiella pneumoniae


<220>
<221>  misc_feature
<222>  (420)..(420)
<223>  n is t or a

<220>
<221>  misc_feature
<222>  (456)..(456)
<223>  n is c or t

<400>  28
atgactatca aagtaggtat caacggtttt ggccgtatcg gtcgcattgt tttccgtgct      60

gctcagaaac gttctgacat cgagatcgtt gcaatcaacg acctgttaga cgcagagtac     120

atggcttaca tgctgaagta tgactccact cacggtcgtt tcgacggtac cgttgaagtg     180

aaagacggtc atctggtcgt taacggtaaa aaaatccgtg ttaccgctga acgtgacccg     240

gctaacctga agtgggacga agttggtgtt gacgttgttg ctgaagcaac cggtatcttc     300

ctgaccgacg aaaccgctcg taaacacatc accgctggcg cgaaaaaagt cgttctgact     360

ggcccgtcca aagacaacac tccgatgttc gttcgcggcg ctaacttcga cgcttacgcn     420

ggccaggaca tcgtttccaa cgcttcctgc accacnaact gcctggcgcc gctggctaaa     480

gttatcaacg acaacttcgg tatcgttgaa ggcctgatga ccaccgtcca cgctaccacc     540

gctactcaga aaaccgttga tggcccgtct cacaaagact ggcgcggcgg ccgcggcgca     600

gctcagaaca tcatcccgtc ctctaccggc gctgctaaag cagtaggtaa agtactgcca     660

gaactgaacg gcaaactgac cggtatggcg ttccgcgttc cgactccgaa cgtatctgtt     720

gttgacctga ccgttcgtct ggaaaaagca gcgtcctacg aagaaatcaa gaaagccatc 780

aaagccgctt ctgaaggcgc tatgaaaggc gttctgggtt acaccgaaga cgacgttgtt 840

tctaccgact tcaacggcga agtttgcact tccgtgttcg atgctaaagc gggtatcgca 900

ctgaacgaca acttcgtgaa actggtttcc tggtacgaca acgaaaccgg ctactccaac 960

aaagttctgg atctgattgc ccacatctcc aaataa 996

<210> 29
<211> 2691
<212> DNA
<213> Klebsiella pneumoniae

<220>
<221> misc_feature
<222> (1608)..(1608)
<223> n is c or t

<400> 29
atgacagatg tgactattaa agcgctggcc tcagagattc agacctctgt ggatcgcctg 60

atacagcaat ttgctgacgc aggcatccgc aaatcggctg atgattctgt gacctcgcaa 120

gagaaacaaa ctttgttgac gcacctgaac cgtgaacacg gctcggcgcc agacaagctg 180

acgttacagc gtaagacgcg cagtacgttg aatattccag gtaccggtgg aaagagtaaa 240

tcggtacaaa tcgaagtccg caagaaacgc acctttgtga aacgcgatcc gcaagaggct 300

gaacgcctgg ccgcggaaga gcaggcgcag cgtgaagcgg aagagcaggc ccgtcgtgaa 360

gctgaagaag cagcgaaacg cgaggcgcaa ttaaaagctg aacgtgaggc cgcagaacaa 420

gctaaacgtg aagtcgctga taaagcgaaa cgtgaagctg cggaaaaaga caaagtgagc 480

aatcaacata ccgacgaaat gaccaaaacc gcccaggctg aaaagatccg tcgcgagaac 540

gaagccgcgg aattgaagcg caaatcggaa gaagaagcac gccgcaaact tgaagaagaa 600

gcgcgccgtg tagcggaaga agcacgccgt atggctgaag aaaacgaaaa aaattggtct 660

gaaacctcag acagcccgga agatagcagc gactatcacg tcaccacatc acagcatgct 720

cgtcaggctg aagatgataa cgatcgtgaa gtcgaaggcg gtcgcggccg tagccgtagc 780

agcaaagcgg ctcgtccggc gaagaaaggc aacaaacacg ctgaatcgaa agctgatcgt 840

gaagaagccc gcgcggccgt tcgcggcggt aaaggcggta agcaccgtaa aggttccgct 900

ctgcagcagg gcttccagaa gccagcgcag gccgttaacc gtgacgtcgt aatcggcgaa 960

accatcaccg ttggcgaact ggctaacaag atggcggtga aaggttctca ggtcatcaaa 1020

gcgatgatga agctgggcgc catggcgacc atcaaccagg tcatcgacca ggaaaccgca 1080

cagctggttg ccgaagagat gggccacaaa gttatcctgc gtcgtgaaaa cgaactggaa 1140

gaagccgtaa tgagcgaccg tgacaccggc gcggcggctg aaccgcgcgc accggtcgtg 1200

accattatgg gtcacgttga ccacggtaaa acctcgctgc tggactacat tcgttctacc 1260

aaggttgcct ccggcgaagc gggtggtatt acccagcaca tcggtgctta ccacgtcgaa 1320

accgacaacg gcatgatcac cttcctggat accccgggcc acgccgcgtt tacctccatg 1380

cgtgctcgtg gcgcgcaggc gacggatatc gtggttctgg tggtggcggc agacgacggc 1440

gtgatgccgc agactatcga agctatccag cacgctaaag cggcgcaggt accggtggta 1500

gtggcggtga acaagatcga taagccagaa gccgatccgg atcgcgtgaa gaacgaactg 1560

tcccagtacg gcatcctgcc ggaagagtgg ggcggcgaga gccagttngt ccacgtttcc 1620

gcgaaagcgg gtaccggcat cgacgacctg ctggacgcga tcctgctgca ggctgaagtt 1680

cttgagctga aagcggtccg caacggtatg gcgagcggcg cggtcatcga atccttcctt 1740

gataaaggtc gtggtccggt agctaccgtt ctggttcgcg aaggtactct gcacaagggc 1800

gacattgttc tgtgcggctt cgaatatggc cgtgtgcgcg cgatgcgtga cgaactgggt 1860

cgcgaagtgc tggaagcggg tccgtccatt ccggtggaaa tcctcggcct gtccggtgtg 1920

ccggctgccg gtgatgaagt gaccgtagtg cgtgacgaga aaaaagcgcg tgaagtggcg 1980

ctgtatcgtc agggcaaatt ccgtgaagtt aagctggcgc gtcagcagaa atctaaactg 2040

gaaaacatgt tcgctaacat gaccgaaggc gaagttcacg aagtgaacat cgtactgaaa 2100

gcggacgtac agggttctgt cgaagcgatt tccgattcct tactgaaact gtctaccgac 2160

gaagtgaaag tgaagatcat cggttccggc gtaggtggta tcaccgaaac cgacgctacc 2220

ctggcagcag catccaacgc gattctggtt ggcttcaacg ttcgtgccga tgcctctgcg 2280

cgtaaagtta tcgaagcgga aagcctggat ctgcgttact actccgtcat ctataacctg 2340

atcgacgaag tgaaagcggc gatgagcggc atgctgtctc cggaactgaa acagcagatc 2400

atcggtctgg ctgaagtgcg tgatgtcttc aaatcgccga aattcggcgc catcgcgggc 2460

tgtatggtta ccgaagggac gatcaaacgt cacaacccaa tccgcgttct gcgtgacaac 2520

gtggttatct atgaaggcga gctggaatcc ctgcgccgct tcaaagatga cgttaacgaa 2580

gtccgtaacg gcatggaatg tggtatcggt gtgaagaact acaacgacgt tcgcgttggc 2640

gatatgatcg aagtgttcga aatcatcgaa atccagcgta gcatcgatta a 2691

<210> 30
<211> 16
<212> DNA
<213> Artificial Sequence

<220>
<223> Primer Sequence

<220>
<221> misc_feature
<222> (5)..(5)

<223>  n is inosine

<220>
<221>  modified_base
<222>  (5)..(5)
<223>  I

<400>  30
ccgcntcctg caccac                                                        16


<210>  31
<211>  30
<212>  DNA
<213>  Artificial Sequence

<220>
<223>  Primer Sequence

<400>  31
acgttggatg tgaagaacga actgtcccag                                         30


<210>  32
<211>  29
<212>  DNA
<213>  Artificial Sequence

<220>
<223>  Primer Sequence

<400>  32
acgttggatg gtccagcagg tcgtcgatg                                          29


<210>  33
<211>  20
<212>  DNA
<213>  Artificial Sequence

<220>
<223>  Primer Sequence

<400>  33
gtgtgcggcg agagccagtt                                                    20


<210>  34
<211>  30
<212>  DNA
<213>  Artificial Sequence

<220>
<223>  Primer Sequence

<400>  34
acgttggatg gggtcaccat tctgccttta                                         30


<210>  35
<211>  29
<212>  DNA
<213>  Artificial Sequence

<220>
<223>  Primer Sequence

<400>  35
acgttggatg cgcccgcttt cgcttccac                                          29


<210>  36
<211>  21
<212>  DNA
<213>  Artificial Sequence

<220>
<223>  Primer Sequence


<220>
<221>  misc_feature
<222>  (10)..(10)
<223>  n is inosine

<220>
<221>  modified_base
<222>  (10)..(10)
<223>  I

<220>
<221>  misc_feature
<222>  (13)..(13)
<223>  n is inosine

<220>
<221>  modified_base
<222>  (13)..(13)
<223>  I

<400>  36
aagtattcan aangccggta c                                                  21


<210>  37
<211>  30
<212>  DNA
<213>  Artificial Sequence

<220>
<223>  Primer Sequence

<400>  37
acgttggatg acaatatcta cctggcgacc                                         30


<210>  38
<211>  30
<212>  DNA
<213>  Artificial Sequence

<220>
<223>  Primer Sequence

<400>  38
acgttggatg gttgaagtag taggtcaggc                                         30

<210>  39
<211>  16
<212>  DNA
<213>  Artificial Sequence

<220>
<223>  Primer Sequence

<400>  39
ggctgatcgg ggtcat                                                        16


<210>  40
<211>  29
<212>  DNA
<213>  Artificial Sequence

<220>
<223>  Primer Sequence

<400>  40
acgttggatg tccctcggct atgtgctgt                                          29


<210>  41
<211>  30
<212>  DNA
<213>  Artificial Sequence

<220>
<223>  Primer Sequence

<400>  41
acgttggatg cgaaggcgtt catgtttttg                                         30


<210>  42
<211>  17
<212>  DNA
<213>  Artificial Sequence

<220>
<223>  Primer Sequence


<220>
<221>  misc_feature
<222>  (6)..(6)
<223>  n is inosine

<220>
<221>  modified_base
<222>  (6)..(6)
<223>  I

<400>  42
gatatngaag gggtggg                                                       17


<210>  43
<211>  30

<212>  DNA
<213>  Artificial Sequence

<220>
<223>  Primer Sequence

<400>  43
acgttggatg tgattaactc cctgtccgtg                                        30


<210>  44
<211>  30
<212>  DNA
<213>  Artificial Sequence

<220>
<223>  Primer Sequence

<400>  44
acgttggatg ccttcttcga tagcagacag                                        30


<210>  45
<211>  22
<212>  DNA
<213>  Artificial Sequence

<220>
<223>  Primer Sequence


<220>
<221>  misc_feature
<222>  (4)..(4)
<223>  n is inosine

<220>
<221>  modified_base
<222>  (4)..(4)
<223>  I

<400>  45
ggcnccgtat cgtaaagtga cc                                                22


<210>  46
<211>  20
<212>  DNA
<213>  Artificial Sequence

<220>
<223>  Primer Sequence

<400>  46
ctattgtgtt ggccgtctac                                                   20


<210>  47
<211>  20
<212>  DNA
<213>  Artificial Sequence

<220>

<223>  Primer Sequence

<400>  47
aatccctcga gcgcgagtct                                                    20


<210>  48
<211>  20
<212>  DNA
<213>  Artificial Sequence

<220>
<223>  Primer Sequence

<400>  48
ccaatattat gcacccggtc                                                    20


<210>  49
<211>  20
<212>  DNA
<213>  Artificial Sequence

<220>
<223>  Primer Sequence

<400>  49
gagctggcgg aaaaccagat                                                    20


<210>  50
<211>  15
<212>  DNA
<213>  Artificial Sequence

<220>
<223>  Primer Sequence

<400>  50
ctgagcgggt gcatg                                                         15


<210>  51
<211>  20
<212>  DNA
<213>  Artificial Sequence

<220>
<223>  Primer Sequence

<400>  51
atgttggtgt tcgtttccgc                                                    20


<210>  52
<211>  20
<212>  DNA
<213>  Artificial Sequence

<220>
<223>  Primer Sequence

<400>  52

gtttaaaaga cgagcacgga                                                    20

<210>  53
<211>  15
<212>  DNA
<213>  Artificial Sequence

<220>
<223>  Primer Sequence

<400>  53
gtaatggcgt ctgcg                                                         15

<210>  54
<211>  19
<212>  DNA
<213>  Artificial Sequence

<220>
<223>  Primer Sequence

<400>  54
gagaataagc agcaaggat                                                     19

<210>  55
<211>  20
<212>  DNA
<213>  Artificial Sequence

<220>
<223>  Primer Sequence

<400>  55
ccatcccact taaagacttg                                                    20

<210>  56
<211>  20
<212>  DNA
<213>  Artificial Sequence

<220>
<223>  Primer Sequence

<400>  56
gcgggtaaaa atgcttggtt                                                    20

<210>  57
<211>  20
<212>  DNA
<213>  Artificial Sequence

<220>
<223>  Primer Sequence

<400>  57
gtttcgtctg gatcgcactg                                                    20

<210>  58
<211>  19
<212>  DNA
<213>  Artificial Sequence

<220>
<223>  Primer Sequence

<400>  58
tgggtttcgc ccagcgcat                                                    19


<210>  59
<211>  15
<212>  DNA
<213>  Artificial Sequence

<220>
<223>  Primer Sequence

<400>  59
agagacacca ccacg                                                        15


<210>  60
<211>  20
<212>  DNA
<213>  Artificial Sequence

<220>
<223>  Primer Sequence

<400>  60
gcgctacagt acagcgataa                                                   20


<210>  61
<211>  19
<212>  DNA
<213>  Artificial Sequence

<220>
<223>  Primer Sequence

<400>  61
agacggaacg tttcgtctc                                                    19


<210>  62
<211>  20
<212>  DNA
<213>  Artificial Sequence

<220>
<223>  Primer Sequence

<400>  62
aatgctgaat ggagagccga                                                   20


<210>  63
<211>  20
<212>  DNA

<213>  Artificial Sequence

<220>
<223>  Primer Sequence

<400>  63
ttgaacagca actcaaccag                                                    20


<210>  64
<211>  15
<212>  DNA
<213>  Artificial Sequence

<220>
<223>  Primer Sequence

<400>  64
ggaagcgggg acgga                                                         15


<210>  65
<211>  20
<212>  DNA
<213>  Artificial Sequence

<220>
<223>  Primer Sequence

<400>  65
gatccgatgc tacgagaaag                                                    20


<210>  66
<211>  20
<212>  DNA
<213>  Artificial Sequence

<220>
<223>  Primer Sequence

<400>  66
gtttgaacca tgtacacggc                                                    20


<210>  67
<211>  20
<212>  DNA
<213>  Artificial Sequence

<220>
<223>  Primer Sequence

<400>  67
tggcccgtcc aaagacaaca                                                    20


<210>  68
<211>  20
<212>  DNA
<213>  Artificial Sequence

<220>

<223>  Primer Sequence

<400>  68
atcaggcctt caacgatacc                                                    20


<210>  69
<211>  18
<212>  DNA
<213>  Artificial Sequence

<220>
<223>  Primer Sequence

<400>  69
ggaaacgatg tcctggcc                                                      18


<210>  70
<211>  939
<212>  DNA
<213>  Klebsiella pneumoniae


<220>
<221>  misc_feature
<222>  (633)..(633)
<223>  n is t or c

<400>  70
atgaaagttg cagtccttgg cgctgccggt ggtatcggcc aggcgcttgc cctactactt      60

aagacccaac tgccttcagg ttcagagctc tcgttgtacg acatcgctcc ggttacgccg     120

ggcgtggcgg tagatctaag tcatatcccc acagatgtaa aaattaaagg attttccggt     180

gaagacgcta ctccggcgct ggaaggcgcg gatgtagtgc tgatctccgc gggcgtggcg     240

cgtaagcccg gcatggatcg ttccgacctg tttaatgtga atgcgggtat cgtgaagaac     300

ctcgtgcagc agattgccaa aacctgcccg caggcctgca tcggcattat caccaacccg     360

gtgaatacca ccgtggctat cgccgccgaa gtactgaaaa aagccggcgt gtacgataaa     420

aacaaactgt tcggcgttac cacgctggac atcatccgtt ccaatacctt tgtggcggag     480

ctgaaaggta aatcggcaac cgaggtggaa gtcccggtca ttggtggtca ctccggggtc     540

accattctgc ctttactgtc gcagatcccc ggcgtcagct ttagcgatca ggaaattgcc     600

gacctgacta aacgtattca gaacgccggt acngaagtcg tggaagcgaa agcgggcggc     660

gggtcggcga ccttgtcgat gggccaggcg gctgcccgtt ttggtctctc tctggttcgc     720

gccatgcagg gggaaaaagg cgtggtggag tgcgcctacg tggaaggcga cggccactat     780

gcgcgtttct tctcccagcc gctgctgctg ggaaaaaacg gcgtggaaga gcgacagtct     840

atcggcaaac tcagcgcctt tgaacagcag gcgctggagg gcatgctgga caccctgaag     900

aaagatatcg cgctcggcga agacttcgtc aacaagtaa                            939

<210>  71
<211>  1029
<212>  DNA
<213>  Klebsiella pneumoniae


<220>
<221>  misc_feature
<222>  (732)..(732)
<223>  n is g or a

<220>
<221>  misc_feature
<222>  (867)..(867)
<223>  n is g or a

<400>  71
atgatgatgg gctttgtggc ttcaacagcg acgcaggcag cggaagttta taataagaac      60

gcgaacaagc tggatgtgta cggcaagatc aaagccatgc actatttcag cgactatgac     120

agcaaggatg gcgatcagac ctacgtgcgt ttcggtatta aaggcgaaac gcagattaac     180

gacgacctga ccggctatgg ccgttgggaa tctgaattct ccggtaacaa aaccgagagc     240

gactccagcc agaaaacccg tctggcgttc gccggcgtga agctgaagaa ctacggctcc     300

ttcgattacg gtcgtaacct tggcgccctg tacgacgtgg aagcctggac cgatatgttc     360

ccggaattcg gcggcgattc ctctgcccag accgataact ttatgaccaa gcgcgccagc     420

ggcctggcga cctaccgcaa caccgacttc ttcggtctgg tggatggcct ggatctgacc     480

ctgcagtacc agggtaaaaa cgaaggccgt gaagcgaaga aacagaacgg cgacggcgtc     540

ggcacctcgt taagctatga tttcggcggc agcgacttcg ccgtcagcgc agcctacacc     600

agctccgacc gtaccaacga tcagaacctg ctggcccgcg gccagggttc gaaagcggaa     660

gcctgggcga ccggcctgaa atatgacgcc aacaatatct acctggcgac catgtactct     720

gaaacccgca anatgacccc gatcagcggc ggctttgcca acaaagcgca gaactttgaa     780

gcggtggcgc agtatcagtt cgacttcggt ctgcgtccgt ccctcggcta tgtgctgtcg     840

aaagggaagg atatcgaagg ggtgggnagt gaagatctgg ttaactacat cgacgtgggc     900

ctgacctact acttcaacaa aaacatgaac gccttcgtgg attacaaaat caaccagctg     960

aaaagcgata acaaactcgg catcaacgat gacgacatcg tcgcgctggg tatgacctac    1020

cagttctga                                                            1029


<210>  72
<211>  15
<212>  DNA
<213>  Artificial Sequence

<220>
<223>  Primer Sequence

<220>
<221>  misc_feature
<222>  (4)..(4)
<223>  n is inosine

<220>
<221>  modified_base
<222>  (4)..(4)
<223>  I

<400>  72
cgcntcctgc accac                                                        15


<210>  73
<211>  20
<212>  DNA
<213>  Artificial Sequence

<220>
<223>  Primer Sequence

<400>  73
tgaagaacga actgtcccag                                                   20


<210>  74
<211>  19
<212>  DNA
<213>  Artificial Sequence

<220>
<223>  Primer Sequence

<400>  74
gtccagcagg tcgtcgatg                                                    19


<210>  75
<211>  16
<212>  DNA
<213>  Artificial Sequence

<220>
<223>  Primer Sequence

<400>  75
gcggcgagag ccagtt                                                       16


<210>  76
<211>  20
<212>  DNA
<213>  Artificial Sequence

<220>
<223>  Primer Sequence

<400>  76
gggtcaccat tctgccttta                                                   20


<210>  77

<211>  19
<212>  DNA
<213>  Artificial Sequence

<220>
<223>  Primer Sequence

<400>  77
cgcccgcttt cgcttccac                                                    19


<210>  78
<211>  17
<212>  DNA
<213>  Artificial Sequence

<220>
<223>  Primer Sequence


<220>
<221>  misc_feature
<222>  (6)..(6)
<223>  n is inosine

<220>
<221>  modified_base
<222>  (6)..(6)
<223>  I

<220>
<221>  misc_feature
<222>  (9)..(9)
<223>  n is inosine

<220>
<221>  modified_base
<222>  (9)..(9)
<223>  I

<400>  78
attcanaang ccggtac                                                      17


<210>  79
<211>  20
<212>  DNA
<213>  Artificial Sequence

<220>
<223>  Primer Sequence

<400>  79
acaatatcta cctggcgacc                                                   20


<210>  80
<211>  20
<212>  DNA
<213>  Artificial Sequence

<220>
<223>  Primer Sequence

<400>  80
gttgaagtag taggtcaggc                                                   20


<210>  81
<211>  15
<212>  DNA
<213>  Artificial Sequence

<220>
<223>  Primer Sequence

<400>  81
gctgatcggg gtcat                                                        15


<210>  82
<211>  19
<212>  DNA
<213>  Artificial Sequence

<220>
<223>  Primer Sequence

<400>  82
tccctcggct atgtgctgt                                                    19


<210>  83
<211>  20
<212>  DNA
<213>  Artificial Sequence

<220>
<223>  Primer Sequence

<400>  83
cgaaggcgtt catgtttttg                                                   20


<210>  84
<211>  20
<212>  DNA
<213>  Artificial Sequence

<220>
<223>  Primer Sequence

<400>  84
tgattaactc cctgtccgtg                                                   20


<210>  85
<211>  20
<212>  DNA
<213>  Artificial Sequence

<220>
<223>  Primer Sequence

<400>  85
ccttcttcga tagcagacag                                                   20

<210>  86
<211>  20
<212>  DNA
<213>  Artificial Sequence

<220>
<223>  Primer Sequence


<220>
<221>  misc_feature
<222>  (2)..(2)
<223>  n is inosine

<220>
<221>  modified_base
<222>  (2)..(2)
<223>  I

<400>  86
cnccgtatcg taaagtgacc                                                    20


<210>  87
<211>  882
<212>  DNA
<213>  Klebsiella pneumoniae


<220>
<221>  misc_feature
<222>  (814)..(814)
<223>  n is c or t

<400>  87
atgtcactgt atcgccgtct agttctgctg tcttgtctct catggccgct ggctggcttt      60

tctgccaccg cgctgaccaa cctcgtcgcg gaaccattcg ctaaactcga acaggacttt     120

ggcggctcca tcggtgtgta cgcgatggat accggctcag gcgcaactgt aagttaccgc     180

gctgaggagc gcttcccact gtgcagctca ttcaagggct ttcttgctgc cgctgtgctg     240

gctcgcagcc agcagcaggc cggcttgctg gacacaccca tccgttacgg caaaaatgcg     300

ctggttccgt ggtcacccat ctcggaaaaa tatctgacaa caggcatgac ggtggcggag     360

ctgtccgcgg ccgccgtgca atacagtgat aacgccgccg ccaatttgtt gctgaaggag     420

ttgggcggcc cggccgggct gacggccttc atgcgctcta tcggcgatac cacgttccgt     480

ctggaccgct gggagctgga gctgaactcc gccatcccag gcgatgcgcg cgatacctca     540

tcgccgcgcg ccgtgacgga aagcttacaa aaactgacac tgggctctgc actggctgcg     600

ccgcagcggc agcagtttgt tgattggcta aagggaaaca cgaccggcaa ccaccgcatc     660

cgcgcggcgg tgccggcaga ctgggcagtc ggagacaaaa ccggaacctg cggagtgtat     720

ggcacggcaa atgactatgc cgtcgtctgg cccactgggc gcgcacctat tgtgttggcc     780

gtctacaccc gggcgcctaa caaggatgac aagnacagcg aggccgtcat cgccgctgcg 840

gctagactcg cgctcgaggg attgggcgtc aacgggcagt aa 882

<210> 88
<211> 813
<212> DNA
<213> Klebsiella pneumoniae

<220>
<221> misc_feature
<222> (82)..(82)
<223> n is c or g

<400> 88
atggaattgc ccaatattat gcacccggtc gcgaagctga gcaccgcatt agccgctgca 60

ttgatgctga gcgggtgcat gnccggtgaa atccgcccga cgattggcca gcaaatggaa 120

actggcgacc aacggtttgg cgatctggtt ttccgccagc tcgcaccgaa tgtctggcag 180

cacacttcct atctcgacat gccgggtttc ggggcagtcg cttccaacgg tttgatcgtc 240

agggatggcg gccgcgtgct ggtggtcgat accgcctgga ccgatgacca gaccgcccag 300

atcctcaact ggatcaagca ggagatcaac ctgccggtcg cgctggcggt ggtgactcac 360

gcgcatcagg acaagatggg cggtatggac gcgctgcatg cggcggggat tgcgacttat 420

gccaatgcgt tgtcgaacca gcttgccccg caagagggga tggttgcggc gcaacacagc 480

ctgactttcg ccgccaatgg ctgggtcgaa ccagcaaccg cgcccaactt tggcccgctc 540

aaggtatttt accccggccc cggccacacc agtgacaata tcaccgttgg gatcgacggc 600

accgacatcg cttttggtgg ctgcctgatc aaggacagca aggccaagtc gctcggcaat 660

ctcggtgatg ccgacactga gcactacgcc gcgtcagcgc gcgcgtttgg tgcggcgttc 720

cccaaggcca gcatgatcgt gatgagccat tccgcccccg atagccgcgc cgcaatcact 780

catacggccc gcatggccga caagctgcgc tga 813

<210> 89
<211> 825
<212> DNA
<213> Klebsiella pneumoniae

<400> 89
atgaaaaaaa ttttgctgct gcatatgttg gtgttcgttt ccgccactct cccaatcagt 60

tccgtggctt ctgatgaggt tgaaacgctt aaatgcacca tcatcgcaga cgccattacc 120

ggaaatacct tatatgagac cggagaatgt gcccgtcgtg tgtctccgtg ctcgtctttt 180

aaacttccat tggcaatcat ggggtttgat agtggaatct tgcagtcgcc aaaatcacct 240

acgtgggaat tgaagccgga atacaacccg tctccgagag atcgcacata caaacaagtc 300

tatccggcgc tatggcaaag cgactctgtt gtctggttct cgcagcaatt aacaagccgt 360

ctgggagttg atcggttcac ggaatacgta aagaaatttg agtacggtaa tcaagatgtt 420

tccggtgact cggggaagca taacggcttg acccagtcat ggctgatgtc gtcgctcacc 480

atatctccca aggagcaaat tcagtttctt ctacgctttg tcgcgcataa gctgcctgta 540

tccgaagcgg cttatgacat ggcgtatgcc acaatcccgc agtaccaggc agccgaagga 600

tgggctgtac atggaaaaag cggcagcggc tggcttcggg acaataacgg caagataaat 660

gaaagtcgtc cgcagggctg gttcgtgggc tgggctgaaa aaaacggacg gcaagttgtt 720

ttcgcccgat tggaaatagg aaaggaaaag tccgatattc ccggcgggtc taaagcacga 780

gaggatattc tcgtggaatt acccgtgttg atgggtaaca aatga 825

<210> 90
<211> 798
<212> DNA
<213> Klebsiella pneumoniae

<220>
<221> misc_feature
<222> (186)..(186)
<223> n is g or a

<400> 90
atgcgtgtat tagccttatc ggctgtgttt ttggtggcat cgattatcgg aatgcctgcg 60

gtagcaaagg aatggcaaga aaacaaaagt tggaatgctc actttactga acataaatca 120

cagggcgtag ttgtgctctg gaatgagaat aagcagcaag gatttaccaa taatcttaaa 180

cgggcnaacc aagcattttt acccgcatct acctttaaaa ttcccaatag cttgatcgcc 240

ctcgatttgg gcgtggttaa ggatgaacac caagtcttta agtgggatgg acagacgcgc 300

gatatcgcca cttggaatcg cgatcataat ctaatcaccg cgatgaaata ttcagttgtg 360

cctgtttatc aagaatttgc ccgccaaatt ggcgaggcac gtatgagcaa gatgctacat 420

gctttcgatt atggtaatga ggacatttcg ggcaatgtag acagtttctg gctcgacggt 480

ggtattcgaa tttcggccac ggagcaaatc agctttttaa gaaagctgta tcacaataag 540

ttacacgtat cggagcgcag ccagcgtatt gtcaaacaag ccatgctgac cgaagccaat 600

ggtgactata ttattcgggc taaaactgga tactcgacta gaatcgaacc taagattggc 660

tggtgggtcg gttgggttga acttgatgat aatgtgtggt tttttgcgat gaatatggat 720

atgcccacat cggatggttt agggctgcgc caagccatca caaaagaagt gctcaaacag 780

gaaaaaatta ttccctag 798

<210> 91
<211> 876
<212> DNA
<213> Escherichia coli

<220>
<221>  misc_feature
<222>  (556)..(556)
<223>  n is c or g

<400>  91
atggtgacaa agagagtgca acggatgatg ttcgcggcgg cggcgtgcat tccgctgctg      60

ctgggcagcg cgccgcttta tgcgcagacg agtgcggtgc agcaaaagct ggcggcgctg     120

gagaaaagca gcggagggcg gctgggcgtc gcgctcatcg ataccgcaga taatacgcag     180

gtgctttatc gcggtgatga acgctttcca atgtgcagta ccagtaaagt tatggcggcc     240

gcggcggtgc ttaagcagag tgaaacgcaa aagcagctgc ttaatcagcc tgtcgagatc     300

aagcctgccg atctggttaa ctacaatccg attgccgaaa aacacgtcaa cggcacaatg     360

acgctggcag agctgagcgc ggccgcgttg cagtacagcg acaataccgc catgaacaaa     420

ttgattgccc agctcggtgg cccgggaggc gtgacggctt ttgcccgcgc gatcggcgat     480

gagacgtttc gtctggatcg cactgaacct acgctgaata ccgccattcc cggcgacccg     540

agagacacca ccacgncgcg ggcgatggca cagacgttgc gtcagcttac gctgggtcat     600

gcgctgggcg aaacccagcg ggcgcagttg gtgacgtggc tcaaaggcaa tacgaccggc     660

gcagccagca ttcgggccgg cttaccgacg tcgtggactg caggtgataa gaccggcagc     720

ggcgactacg gcaccaccaa tgatattgcg gtgatctggc cgcagggtcg tgcgccgctg     780

gttctggtga cctattttac ccagccgcaa cagaacgcag agagccgccg cgatgtgctg     840

gcttcagcgg cgagaatcat cgccgaaggg ctgtaa                               876


<210>  92
<211>  876
<212>  DNA
<213>  Escherichia coli


<220>
<221>  misc_feature
<222>  (453)..(453)
<223>  n is c or t

<400>  92
atggttaaaa aatcactgcg ccagttcacg ctgatggcga cggcaaccgt cacgctgttg      60

ttaggaagtg tgccgctgta tgcgcaaacg gcggacgtac agcaaaaact tgccgaatta     120

gagcggcagt cgggaggcag actgggtgtg gcattgatta acacagcaga taattcgcaa     180

atactttatc gtgctgatga gcgctttgcg atgtgcagca ccagtaaagt gatggccgcg     240

gccgcggtgc tgaagaaaag tgaaagcgaa ccgaatctgt taaatcagcg agttgagatc     300

aaaaaatctg accttgttaa ctataatccg attgcggaaa agcacgtcaa tgggacgatg     360

tcactggctg agcttagcgc ggccgcgcta cagtacagcg ataacgtggc gatgaataag 420

ctgattgctc acgttggcgg cccggctagc gtnaccgcgt tcgcccgaca gctgggagac 480

gaaacgttcc gtctcgaccg taccgagccg acgttaaaca ccgccattcc gggcgatccg 540

cgtgatacca cttcacctcg ggcaatggcg caaactctgc ggaatctgac gctgggtaaa 600

gcattgggcg acagccaacg ggcgcagctg gtgacatgga tgaaaggcaa taccaccggt 660

gcagcgagca ttcaggctgg actgcctgct tcctgggttg tgggggataa aaccggcagc 720

ggtggctatg gcaccaccaa cgatatcgcg gtgatctggc caaaagatcg tgcgccgctg 780

attctggtca cttacttcac ccagcctcaa cctaaggcag aaagccgtcg cgatgtatta 840

gcgtcggcgg ctaaaatcgt caccgacggt ttgtaa 876

<210> 93
<211> 519
<212> DNA
<213> Klebsiella pneumoniae

<220>
<221> misc_feature
<222> (223)..(223)
<223> n is a or c or t

<220>
<221> misc_feature
<222> (454)..(454)
<223> n is t or g

<400> 93
atgactgagc atgaccttgc gatgctctat gagtggctaa atcgatctca tatcgtcgag 60

tggtggggcg gagaagaagc acgcccgaca cttgctgacg tacaggaaca gtacttgcca 120

agcgttttag cgcaagagtc cgtcactcca tacattgcaa tgctgaatgg agagccgatt 180

gggtatgccc agtcgtacgt tgctcttgga agcggggacg ganggtggga agaagaaacc 240

gatccaggag tacgcggaat agaccagtta ctggcgaatg catcacaact gggcaaaggc 300

ttgggaacca agctggttcg agctctggtt gagttgctgt tcaatgatcc cgaggtcacc 360

aagatccaaa cggacccgtc gccgagcaac ttgcgagcga tccgatgcta cgagaaagcg 420

gggtttgaga ggcaaggtac cgtaaccacc ccanatggtc cagccgtgta catggttcaa 480

acacgccagg cattcgagcg aacacgcagt gatgcctaa 519

<210> 94
<211> 4062
<212> DNA
<213> Klebsiella pneumoniae

<220>
<221> misc_feature

<222>   (1819)..(1819)
<223>   n is a or g

<400>   94
atgggtcgac ttgtcagcga gctgaggaac cctatggttt actcctatac cgagaaaaaa      60

cgtattcgta aggattttgg taaacgtcca caagttctgg atgtgccata tctcctttct     120

atccagcttg actcgttcca gaagtttatc gagcaagatc ctgaagggca gtatggtctg     180

gaagctgctt tccgttccgt attcccgatt cagagctaca gcggtaattc cgagctgcaa     240

tacgtcagct accgtctggg cgaaccggtt tttgacgtta aagaatgtca gatccgtggc     300

gtgacttatt ctgcaccgct gcgcgtaaaa ctgcgtctgg tgatctacga gcgcgaagcg     360

ccagaaggca ccgtcaaaga cattaaagaa caagaagtct acatgggcga aattccgctc     420

atgaccgaca acggtacctt tgttatcaac ggtaccgagc gtgttatcgt ttctcagctg     480

caccgtagcc cgggcgtctt ctttgacagt gacaaaggta agacccactc ctccggtaag     540

gttctgtata acgcacgtat catcccttac cgcggttcct ggctggactt cgaattcgac     600

ccgaaagaca acctgttcgt tcgtatcgac cgtcgtcgta agctgccggc taccatcatc     660

ctgcgcgcgc tgaattacac cactgagcag atccttgacc tgttcttcga aaaagtggtc     720

tttgaaattc gcgacaacaa gctgcagatg gagctgattc cggagcgtct gcgcggtgaa     780

actgcctcct tcgatatcga agccaacggc aaagtgtacg ttgagaaagg ccgtcgcatc     840

acggctcgtc atatccgtca gctggaaaaa gacgatatca agcatatcga agtgccggtt     900

gaatatatcg cgggcaaagt ggcggctaaa gactatatcg atgaagcgac tggcgaactg     960

atctgcccgg cgaacatgga gctgagcctg gatctgctgg ctaagctgag ccagtctggc    1020

cacaagcgta tcgaaacgct gttcaccaac gatctggacc acggcccgta catctctgaa    1080

acggtacgcg tcgacccgac taacgatcgt ctgagcgcgc tggtagaaat ctaccgcatg    1140

atgcgccctg gcgagccgcc gactcgtgaa gccgctgaaa gtctgtttga gaacctgttc    1200

ttctctgaag accgctacga tctgtccgcg gttggtcgta tgaagttcaa ccgttctctg    1260

ctgcgcgacg aaatcgaagg ttccggtatc ctgagcaaag acgacatcat cgaagtgatg    1320

aagaagctca tcgatatccg taacggtaaa ggcgaagtgg acgatatcga ccacctcggc    1380

aaccgtcgta tccgttccgt tggcgaaatg gcggaaaacc agttccgcgt tggcctggta    1440

cgtgtagagc gtgcggtgaa agagcgtctg tctcttggcg atctggatac cctgatgcct    1500

caggatatga tcaacgccaa gccgatttcc gcagcagtga aagagttctt tggttccagc    1560

cagctgtctc agtttatgga ccagaacaac ccgctgtctg agattacgca caaacgtcgt    1620

atctccgcac tcggcccagg cggtctgacc cgtgagcgcg caggcttcga agttcgagac    1680

gtacacccga cccactacgg tcgcgtatgt ccgatcgaaa cgcctgaagg tccaaacatc    1740

ggtctgatta actccctgtc cgtgtacgcg cagaccaacg aatatggctt ccttgagacg    1800

ccgtatcgta aagtgaccna cggtgtggtt actgacgaaa ttcactacct gtctgctatc 1860

gaagaaggca actacgttat cgctcaggcg aactccaacc tggatgaaaa cggccacttc 1920

gtagaagatc tggttacctg ccgtagcaaa ggcgaatcca gcttgttcag ccgcgaccag 1980

gttgactaca tggacgtatc cacccagcag gtggtatccg tcggtgcgtc cctgatcccg 2040

ttcctggaac acgatgacgc caaccgtgca ttgatgggtg cgaacatgca acgtcaggcg 2100

gttccgactc tgcgcgctga taagccgctg gttggtaccg gtatggaacg tgctgttgcc 2160

gttgactccg gtgttactgc cgtggctaaa cgtggcggta ccgttcagta cgtggatgct 2220

tcccgtatcg ttatcaaagt taacgaagac gagatgtacc cgggcgaagc aggtatcgac 2280

atctataacc tgaccaagta cacccgttct aaccagaaca cctgcatcaa ccagatgcct 2340

tgcgtgtccc tgggcgaacc tattgagcgc ggcgacgtgc tggcagacgg cccgtccacc 2400

gacctcggtg agctggcgct gggtcagaac atgcgtgtag cgttcatgcc gtggaacggt 2460

tacaacttcg aagactccat cctcgtttcc gagcgtgttg tccaggaaga ccgtttcacc 2520

actatccaca ttcaggaact ggcgtgcgtg tcccgtgaca ccaagctggg gccggaagag 2580

atcaccgctg acatcccgaa cgtgggtgaa gctgcgctct ccaaactgga tgaatccggt 2640

atcgtttata tcggtgcgga agtgaccggc ggcgacattc tggttggtaa ggtaacgccg 2700

aaaggtgaaa cccagctgac gccggaagag aaactgctgc gcgcgatctt cggtgagaaa 2760

gcgtctgatg ttaaagactc ttctctgcgc gtaccgaacg gtgtctctgg taccgttatc 2820

gacgttcagg tctttacccg tgacggcgta gaaaaagaca aacgtgctct ggaaatcgaa 2880

gagatgcagc tgaagcaggc caaaaaagac ctgtctgaag aactgcagat cctcgaagcc 2940

ggtctgttca gtcgtatcta cgcggtgctg gtctccggtg gcgttgaagc tgagaagctc 3000

gacaagctgc ctcgcgatcg ctggctggaa ctgggcctga ccgacgaaga gaaacaaaat 3060

cagctggaac agctggctga gcagtatgac gaactgaaac acgagttcga gaaaaaactc 3120

gaagcgaaac gccgtaaaat cacccaaggc gacgatctgg caccgggcgt gctgaagatt 3180

gttaaggtat atctggccgt taaacgccgt atccagcctg gtgataagat ggcaggtcgt 3240

cacggtaaca agggtgttat ctctaagatc aacccgatcg aagatatgcc gcacgatgct 3300

aacggtacgc cggtagatat cgtactgaac ccgctgggcg taccgtctcg tatgaacatc 3360

ggtcagatcc ttgaaactca cctgggtatg gctgcaaaag gcatcggcga taagatcaac 3420

gccatgctga agcagcagca ggaagtcgcg aaactgcgcg aattcatcca gcgtgcttac 3480

gatctgggcg ctgacgttcg ccagaaagtt gacctgaata ccttcagcga tgaagaagtt 3540

ctgcgtctgg ctgaaaacct gcgtaaaggc atgccgatcg caacgccggt attcgacggt 3600

gcgaaagaag ctgaaatcaa agagctgctg cagctgggcg acctgccgac ttccggtcag 3660

atcaccctgt ttgatggccg caccggtgaa cagttcgaac gtccggtaac cgtgggttac 3720

atgtacatgc tgaaactgaa ccacctggtc gacgacaaaa tgcacgctcg ttccaccggt 3780

tcttacagcc tggttactca gcagccgctg ggtggtaagg cacagttcgg tggtcagcgc 3840

ttcggtgaga tggaagtgtg ggcgctggaa gcatacggcg cggcctacac cctgcaggaa 3900

atgctcaccg ttaagtctga tgacgtgaac ggtcgtacca agatgtataa aaacatcgtg 3960

gacggcaatc accagatgga accgggcatg ccggaatcct tcaacgtact gttgaaagaa 4020

attcgctcgc tgggcatcaa catcgaactg gaagacgagt aa 4062

## Description

The present invention relates to a molecular method for determining in a bacterial sample both the phylogenetic background and the antibiotic resistances of bacterial strains and to a kit for performing the method.

The responsibility of clinical microbiology laboratories is (i) to identify the bacterial strain, (ii) to perform antibiotic susceptibility testing (AST) and (iii) to provide a surveillance infrastructure. The diagnostic laboratories report to clinicians in the hospitals or to practitioners in order to guide them in their treatment choice or the implementation of hygiene measures.

Today AST involves phenotypic test methods which mostly rely on semi-automated systems. Despite the clinical need, microbiological diagnostics of resistance has experienced little fundamental change over the years. Bacterial antibiotic susceptibility testing still predominantly relies on culture-dependent methods. As a consequence, clinical microbiology is still labor-intensive and slow in comparison to other fields of laboratory medicine. Efforts to decrease the turn-round time of culture for susceptibility testing have been made. Those include the combination of powerful optical systems for growth detection in miniaturized cups and the computerized analysis of growth patterns. Nevertheless, the gain in terms of turn-round time of these new culture-dependent methods remains limited. There are only few molecular resistance profiling assays on the market which provide rapid results in order to guide the clinicians in their treatment choice. However those assays can be applied only on a small selection of pathogens, they do not cover the entire "*Resistome*" of the pathogens and are far too expensive to be introduced into routine diagnostic microbiology. The high price precludes also their broad application for surveillance purposes.

To significantly decrease hospital acquired infections and to prevent outbreaks, it is essential to establish surveillance systems. Management of nosocomial infections, in particular those caused by multidrug-resistant gram negative bacteria, requires a strategy that includes not only the development of new anti-microbial compounds and a rational use of anti-microbial agents, but also early diagnosis for targeted treatment and the implementation of effective infection control principles. In order to do so, in addition to species identification typing of the causing bacterial isolates is essential. This is currently mainly performed by the use of pulse field gel electrophoresis or PCR-based technologies such as e.g. multilocus sequence typing (MLST) assays.

There have been no attempts to use genetic markers of resistance conferring genes for the development of a molecular typing system and to combine resistance typing and genotyping within one molecular method.

In spite of the initial enthusiasm and the huge literature on their diagnostic use, gene-detection-based molecular methods have not yet had the dramatic impact on routine diagnostic microbiology that many predicted. However, the identification of antibiotic susceptibility based on resistance determining molecular markers in combination with the identification of phylogenetic origin may provide the basis for therapeutic advice regarding antibiotic treatment. At the same time, it would provide information on the epidemiologic behavior of this specific isolate. Since cost, morbidity and mortality are significantly lower when phylogenetic information and antibiotic resistances are determined more rapidly, such method would have a great impact on infection management.

In order to meet these challenges the present inventors have established a rapid, cost-effective and robust molecular method. The method is a gene-detection-based high-density molecular method which tests for a plethora of genetic determinants to provide timely information on resistance profiles as well as on phylogenetic origin.

In comparison to other gene-detection-based molecular methods, the method of the present invention has several advantages. As compared to the previously developed molecular methods of resistance testing, the method of the present invention can be applied very broadly to any gram-negative bacterial pathogen as it is not restricted to the detection of specific and species-related genetic determinants. Instead, the method of the present invention comprises a multitude of genetic markers that can be found in various combinations in gram-negative multidrug-resistant bacterial pathogens of diverse species. Furthermore, the method of the present invention may be applied on the MassARRAY (Agena Bioscience) which involves a multiplex PCR coupled with mass spectrometry technology. This technology is robust and rapid and most importantly allows for the detection of many markers at reasonable costs.

Determination of antibiotic resistance by detection of single nucleotide polymorphisms (SNPs) is known from the art. For example US 8,247,170 B2 describes detection of penicillin tolerance in Group B *Streptococcus* via SNPs in the gene encoding penicillin binding protein 4.
US 8,741,563 B2 describes correlation between metronidazole resistance in *Trichomonas vaginalis* and SNPs in tvntr 4 and tvntr 6 genes. US 2012/0009572 A1 describes detection of Methicillin resistance in *Staphylococcus aureus* via SNPs in the gene encoding penicillin binding protein 3. However, all these attempts are limited to detection of resistance of known bacterial species against specific antibiotic drugs. Furthermore, none of these methods provides information on the phylogenetic origin of the bacterial strain.

There is a plethora of resistance conferring genes which are acquired in different combinations by different gram-negative multi-drug resistant isolates. Furthermore those resistance conferring genes exhibit substantial variation in their genomic sequence. The inventors found that if a gene-detection-based high-density molecular system provides information on the presence/absence and the sequence variation of those resistances conferring genes this opens up the unique opportunity to use resistance determinants not only for resistance typing but also for obtaining information on the phylogenetic origin. Moreover, they found that if this resistance determinant phylogenetic information is combined with only a restricted selection of phylogenetic markers, the identification of phylogenetic origin can be even further improved. Information on phylogenetic origin can be used for generating phylogenetic trees and preferably enables sub-classification of the bacterial species. This allows for the detection of transmissions e.g. from patient to patient.

It was therefore an object of the present invention to provide a method that overcomes the problems associated with the prior art methods. The object of the present invention was in particular to derive a high amount of information from a method of low effort. The problem is solved by the subject-matter of the patent claims.

The problem is in particular solved by a method for determining from a bacterial sample information on both the phylogenetic origin and the antibiotic resistances of bacterial strains, the method comprising the steps of:
A) determining which nucleotide is present
   a. at position 814 in the KPC-2 coding sequence (SEQ ID NO:87),
   b. at position 82 in the NDM-1 coding sequence (SEQ ID NO:88),
   c. at position 104 in the OXA-9 coding sequence (SEQ ID NO:89),
   d. at position 186 in the OXA-48 coding sequence (SEQ ID NO:90),
   e. at position 556 in the CTX-M-9 coding sequence (SEQ ID NO:91),
   f. at position 453 in the CTX-M-15 coding sequence (SEQ ID NO:92),
   g. at position 223 in the AAC(6')-lb-cr coding sequence (SEQ ID NO:93), and
   h. at position 454 in the AAC(6')-lb-cr coding sequence (SEQ ID NO:93),
   or at analogous positions in variants or homologous coding sequences having at least 80% sequence identity with the indicated coding sequences, and
B) determining information on both the phylogenetic origin and antibiotic resistances of the bacterial strain based on a comparison of the pattern of the determined nucleotides with respective predetermined patterns of bacterial strain, which phylogenetic origin and antibiotic resistances are known.

Sequence information that can be used for the purpose of comparison is known for a plethora of isolates of many bacterial species and is available in from databases well known to the person skilled in the art.

Preferably, the variants or homologous coding sequences have a sequence identity with the coding sequences as depicted in SEQ ID NOs: 87-93 of at least 90%, more preferably of at least 95%, more preferably of at least 98%, even more preferably of at least 99%.

The term "*% sequence identity*" is well known in the art and is very familiar to the skilled person. Briefly, when a nucleotide sequence is compared by alignment to a sequence as given in the sequence listing of the present invention, the number of nucleotides that are identical in both sequences can be easily identified. The term "*% sequence identity*" describes the ratio of this number of identical nucleotides to the total number of nucleotide residues of the nucleotide sequence of the present invention as given in the sequence listing. In other words, using an alignment, for two sequences the percentage of nucleotide residues that are the same (e.g., 90% or 95% identity) may be determined, when the sequences are compared and aligned for maximum correspondence, for example by using a sequence comparison algorithm as known in the art.

It is preferable that the bacterial strains are multi-drug resistant. In particular, it is advantageous if the bacterial strain contains at least 3, more preferably at least 4, more preferably at least 5, more preferably at least 6 and even more preferably all 7 coding sequences of SEQ ID NOs: 87-93 indicated above.

The bacterial sample that is analyzed with the method of the present invention may be any sample that contains bacterial DNA in an amount sufficient for subsequent analysis. Preferably, the sample is derived from the human or animal body. The sample may for example be a blood sample, a urine sample, a fecal sample, a saliva sample, a sample from a smear test of skin or mucosa or a sample of wound secretion. Preferably, the sample can be easily derived from the human or animal body without greater efforts. In embodiments of the present invention, the above described samples may be further processed by isolation of bacteria and/or bacterial DNA in order to obtain the bacterial sample to be analyzed by the method of the present invention.

Step A) of the method of the present invention may include any processes that enable detection of the nucleotides being present at the indicated positions. The present inventors found that the pattern of single nucleotide polymorphisms (SNPs) at the indicated positions in resistance conferring genes enables determination of information on both the phylogenetic origin and antibiotic resistances of bacterial strains in a surprisingly sensitive manner. Thus, the respective SNP pattern forms a "*molecular fingerprint*" that is extremely useful for early diagnosis of infectious diseases. So far, identification of phylogenetic origin of bacterial isolates was rather focused on SNPs in housekeeping genes. However, the present inventors found that opportunistic bacterial pathogens have acquired distinct sets of resistance conferring genes, a part of which surprisingly exhibits substantial variation to such an extent that obtaining information on phylogenetic origin is enabled by determining the specific SNP pattern of selected positions in those genes. Thus, the pattern of the nucleotides described under step A) above was found to have sufficient discriminative power for distinguishing clonal lineages within species. In addition to selection of suitable therapy, determination of the clonal lineage may also contribute to tracking the course of infection spreading, which may help to optimize infection management and improve hygienic standards.

As such SNPs are universally distributed among multi-drug resistant isolates of many different bacterial species, the method can be excellently applied to all multi-drug resistant bacterial isolates. It is preferable that the bacterial strains are multi-drug resistant. Furthermore, it is preferable, that the bacterial strain is a gram-negative bacterium. In particularly preferred embodiments, the bacterial strain is a multi-drug resistant gram-negative bacterium. More preferably, the genus of the multi-drug resistant bacterial strain is selected from the group consisting of *Klebsiella, Pseudomonas* and *Acinetobacter*. More preferably, the multi-drug resistant bacterial strain belongs to the genus *Klebsiella*. Even more preferably, the multi-drug resistant bacterial strain is a *Klebsiella pneumoniae* strain. In alternative embodiments, the bacterial strain is a gram-positive bacterium.

All genes listed under step A) above are related to antibiotic resistance in bacteria. In particular, KPC-2, NDM-1, OXA-9 and OXA-48 are carbapenemases, CTX-M-9 and CTX-M-15 are beta-lactamases and AAC(6')-lb-cr is an aminoglycoside-modifying enzyme. The SNPs and mutations utilized according to step A) described above are summarized in the following table 1. The nucleotide position is the position of the respective nucleotide in the indicated coding sequences of SEQ ID NOs: 87-93.

**Table 1**

| **Coding Sequence** | **Nucleotide position** | **Most common nucleotide** | **Information** |
|---|---|---|---|
| KPC-2 (SEQ ID NO:87) | 814 | C or T | C indicates KPC-2 group; T indicates KPC-3 group |
| NDM-1 (SEQ ID NO:88) | 82 | C or G | C indicates NDM-1 and others; G indicates NDM-2 |
| OXA-9 (SEQ ID NO:89) | 104 | T | No SNP at this position; rather detection of T indicates presence of OXA-9 gene |
| OXA-48 (SEQ ID NO:90) | 186 | G or A | G indicates OXA-48 and others; A indicates OXA-54 |
| CTX-M-9 (SEQ ID NO:91) | 556 | C or G | C indicates CTX-M-9; G indicates CTX-M-45 |
| CTX-M-15 (SEQ ID NO:92) | 453 | C or T | C indicates CTX-M-15 and others; T indicates CTX-M-10/34/53 |
| AAC(6')-lb-cr (SEQ ID NO:93) | 223 | A or C or T | A or C indicates AAC(6')-lb-cr; T indicates AAC(6')-lb |
| AAC(6')-lb-cr (SEQ ID NO:93) | 454 | T or G | T indicates AAC(6')-lb-cr; G indicates AAC(6')-lb |

Preferably, in order to determine the nucleotides according to step A) of the method, DNA is isolated from the bacterial sample in an initial step. Isolation of DNA from the sample may be performed by any suitable DNA isolation method. Such methods are well known to the skilled person and typically include cell lysis followed by DNA isolation either by DNA precipitation, by phenol-chloroform extraction or by adsorption of DNA to specific DNA binding matrices as for example a silica gel.

Preferably, bacterial DNA comprising the nucleotides of interest is amplified prior to determination of the nucleotides. Preferably, DNA amplification is done by polymerase chain reaction (PCR). Preferably, the PCR is a multiplex PCR, thus enabling amplification of several different DNA sequences simultaneously. The present inventors developed primer sets that are useful for amplification of relevant regions of bacterial DNA with high specificity and sensitivity, particularly in multiplex PCR reactions. Sequences that are preferably comprised by these PCR primers are presented in the sequence listing of the present invention and are summarized in the following table 2. The sequences indicated in table 2 are most preferred. However, variants having a sequence identity of at least 80%, more preferably at least 85% more preferably at least 90%, more preferably at least 94% with the sequences indicated in table 2 are also preferable sequences of the present invention.

**Table 2**

| **Primer name** | **SEQ ID NO:** | **Comprised sequence (5'-3')** |
|---|---|---|
| KPC-2 forward | 46 | CTATTGTGTTGGCCGTCTAC |
| KPC-2 reverse | 47 | AATCCCTCGAGCGCGAGTCT |
| NDM-1 forward | 48 | CCAATATTATGCACCCGGTC |
| NDM-1 reverse | 49 | GAGCTGGCGGAAAACCAGAT |
| OXA-9 forward | 51 | ATGTTGGTGTTCGTTTCCGC |
| OXA-9 reverse | 52 | GTTTAAAAGACGAGCACGGA |
| OXA-48 forward | 54 | GAGAATAAGCAGCAAGGAT |
| OXA-48 reverse | 55 | CCATCCCACTTAAAGACTTG |
| CTX-M-9 forward | 57 | GTTTCGTCTGGATCGCACTG |
| CTX-M-9 reverse | 58 | TGGGTTTCGCCCAGCGCAT |
| CTX-M-15 forward | 60 | GCGCTACAGTACAGCGATAA |
| CTX-M-15 reverse | 61 | AGACGGAACGTTTCGTCTC |
| AAC(6')-lb-cr (223) forward | 62 | AATGCTGAATGGAGAGCCGA |
| AAC(6')-lb-cr (223) reverse | 63 | TTGAACAGCAACTCAACCAG |
| AAC(6')-lb-cr (454) forward | 65 | GATCCGATGCTACGAGAAAG |
| AAC(6')-lb-cr (454) reverse | 66 | GTTTGAACCATGTACACGGC |

By using a combination of corresponding forward and reverse primers, DNA fragments containing the SNP positions of the present invention can be excellently amplified, in particular as PCR products.

In embodiments of the present invention, the amplifying primers do not only comprise the sequences indicated in table 2 or variants having a sequence identity of at least 80%, more preferably at least 85% more preferably at least 90%, more preferably at least 94% therewith but even consist of these sequences. However, the present inventors found that the amplification is advantageously balanced if the amplification primers additionally comprise nucleotides at the 5'-end. Furthermore, this increases the masses of unused primers (out of the mass range on the spectra). Preferably, in addition to the sequences described in table 2 or to variants thereof as indicated above, the amplification primers comprise at least 5, more preferably at least 8, more preferably at least 9, more preferably at least 10 and even more preferably exactly 10 additional nucleotides at the 5'-end. The number of additional nucleotides at the 5'-end should be limited and therefore the amplification primers preferably comprise at most 20, more preferably at most 15, more preferably at most 12, more preferably at most 11 and even more preferably at most 10 additional nucleotides at the 5'-end. Sequences that are comprised in most preferred amplification primers of the present invention are summarized in the following table 3. However, variants having a sequence identity of at least 80%, more preferably at least 85% more preferably at least 90%, more preferably at least 95% with the sequences indicated in table 3 are also preferable sequences of the present invention.

**Table 3**

| **Primer name** | **SEQ ID NO:** | **Comprised sequence (5'-3')** |
|---|---|---|
| KPC-2 long forward | 1 | ACGTTGGATGCTATTGTGTTGGCCGTCTAC |
| KPC-2 long reverse | 2 | ACGTTGGATGAATCCCTCGAGCGCGAGTCT |
| NDM-1 long forward | 4 | ACGTTGGATGCCAATATTATGCACCCGGTC |
| NDM-1 long reverse | 5 | ACGTTGGATGGAGCTGGCGGAAAACCAGAT |
| OXA-9 long forward | 7 | ACGTTGGATGATGTTGGTGTTCGTTTCCGC |
| OXA-9 long reverse | 8 | ACGTTGGATGGTTTAAAAGACGAGCACGGA |
| OXA-48 long forward | 10 | ACGTTGGATGGAGAATAAGCAGCAAGGAT |
| OXA-48 long reverse | 11 | ACGTTGGATGCCATCCCACTTAAAGACTTG |
| CTX-M-9 long forward | 13 | ACGTTGGATGGTTTCGTCTGGATCGCACTG |
| CTX-M-9 long reverse | 14 | ACGTTGGATGTGGGTTTCGCCCAGCGCAT |
| CTX-M-15 long forward | 16 | ACGTTGGATGGCGCTACAGTACAGCGATAA |
| CTX-M-15 long reverse | 17 | ACGTTGGATGAGACGGAACGTTTCGTCTC |
| AAC(6')-lb-cr (223) long forward | 19 | ACGTTGGATGAATGCTGAATGGAGAGCCGA |
| AAC(6')-lb-cr (223) long reverse | 20 | ACGTTGGATGTTGAACAGCAACTCAACCAG |
| AAC(6')-lb-cr (454) long forward | 22 | ACGTTGGATGGATCCGATGCTACGAGAAAG |
| AAC(6')-lb-cr (454) long reverse | 23 | ACGTTGGATGGTTTGAACCATGTACACGGC |

Preferably, the amplifying primers do not only comprise the sequences indicated in table 3 or variants having a sequence identity of at least 80%, more preferably at least 85% more preferably at least 90%, more preferably at least 95% therewith but even consist of these sequences.

The problem of the present invention is also solved by a method for determining from a bacterial sample information on both the phylogenetic origin and the antibiotic resistances of bacterial strains, the method comprising the step of:
A) performing PCR reactions comprising the following primer pairs:
   a. Primer pair 1 consisting of a forward primer comprising a sequence of SEQ ID NO:46 or a sequence having at least 80%, preferably at least 85%, more preferably at least 90%, more preferably at least 94% sequence identity therewith, and a reverse primer comprising a sequence of SEQ ID NO:47 or a sequence having at least 80%, preferably at least 85%, more preferably at least 90%, more preferably at least 94% sequence identity therewith,
   b. Primer pair 2 consisting of a forward primer comprising a sequence of SEQ ID NO:48 or a sequence having at least 80%, preferably at least 85%, more preferably at least 90%, more preferably at least 94% sequence identity therewith, and a reverse primer comprising a sequence of SEQ ID NO:49 or a sequence having at least 80%, preferably at least 85%, more preferably at least 90%, more preferably at least 94% sequence identity therewith,
   c. Primer pair 3 consisting of a forward primer comprising a sequence of SEQ ID NO:51 or a sequence having at least 80%, preferably at least 85%, more preferably at least 90%, more preferably at least 94% sequence identity therewith, and a reverse primer comprising a sequence of SEQ ID NO:52 or a sequence having at least 80%, preferably at least 85%, more preferably at least 90%, more preferably at least 94% sequence identity therewith,
   d. Primer pair 4 consisting of a forward primer comprising a sequence of SEQ ID NO:54 or a sequence having at least 80%, preferably at least 85%, more preferably at least 90%, more preferably at least 94% sequence identity therewith, and a reverse primer comprising a sequence of SEQ ID NO:55 or a sequence having at least 80%, preferably at least 85%, more preferably at least 90%, more preferably at least 94% sequence identity therewith,
   e. Primer pair 5 consisting of a forward primer comprising a sequence of SEQ ID NO:57 or a sequence having at least 80%, preferably at least 85%, more preferably at least 90%, more preferably at least 94% sequence identity therewith, and a reverse primer comprising a sequence of SEQ ID NO:58 or a sequence having at least 80%, preferably at least 85%, more preferably at least 90%, more preferably at least 94% sequence identity therewith,
   f. Primer pair 6 consisting of a forward primer comprising a sequence of SEQ ID NO:60 or a sequence having at least 80%, preferably at least 85%, more preferably at least 90%, more preferably at least 94% sequence identity therewith, and a reverse primer comprising a sequence of SEQ ID NO:61 or a sequence having at least 80%, preferably at least 85%, more preferably at least 90%, more preferably at least 94% sequence identity therewith,
   g. Primer pair 7 consisting of a forward primer comprising a sequence of SEQ ID NO:62 or a sequence having at least 80%, preferably at least 85%, more preferably at least 90%, more preferably at least 94% sequence identity therewith, and a reverse primer comprising a sequence of SEQ ID NO:63 or a sequence having at least 80%, preferably at least 85%, more preferably at least 90%, more preferably at least 94% sequence identity therewith, and
   h. Primer pair 8 consisting of a forward primer comprising a sequence of SEQ ID NO:65 or a sequence having at least 80%, preferably at least 85%, more preferably at least 90%, more preferably at least 94% sequence identity therewith, and a reverse primer comprising a sequence of SEQ ID NO:66 or a sequence having at least 80%, preferably at least 85%, more preferably at least 90%, more preferably at least 94% sequence identity therewith.

All primers of the primer pairs indicated under points a. to h. above may comprise a sequence having at least 80%, more preferably at least 85%, more preferably at least 90%, more preferably at least 94% sequence identity with the sequences of the indicated SEQ ID NOs.

According to the present invention, the PCR reaction of each primer pair may be performed in a separate tube. Thus, 8 separate PCR reactions may be performed. However, it is preferable that the number of separate PCR reactions is reduced in order to save time and resources. Preferably, all PCR reactions are performed in a single tube.

In embodiments of the present invention, the forward and reverse primers do not only comprise the sequences of SEQ ID NOs: 46-49, 51, 52, 54, 55, 57, 58, 60-63, 65, 66 as indicated above or variants having a sequence identity of at least 80%, more preferably at least 85% more preferably at least 90%, more preferably at least 94% therewith but even consist of these sequences. However, the present inventors found that the amplification is advantageously balanced if the forward and reverse primers additionally comprise nucleotides at the 5'-end. Furthermore, this increases the masses of unused primers (out of the mass range on the spectra).

Therefore, a particular preferred method of the present invention for determining from a bacterial sample information on both the phylogenetic origin and the antibiotic resistances of bacterial strains comprises the step of:
A) performing PCR reactions comprising the following primer pairs:
   a. Primer pair 1 consisting of a forward primer comprising a sequence of SEQ ID NO:1 or a sequence having at least 80%, preferably at least 85%, more preferably at least 90%, more preferably at least 95% sequence identity therewith, and a reverse primer comprising a sequence of SEQ ID NO:2 or a sequence having at least 80%, preferably at least 85%, more preferably at least 90%, more preferably at least 95% sequence identity therewith,
   b. Primer pair 2 consisting of a forward primer comprising a sequence of SEQ ID NO:4 or a sequence having at least 80%, preferably at least 85%, more preferably at least 90%, more preferably at least 95% sequence identity therewith, and a reverse primer comprising a sequence of SEQ ID NO:5 or a sequence having at least 80%, preferably at least 85%, more preferably at least 90%, more preferably at least 95% sequence identity therewith,
   c. Primer pair 3 consisting of a forward primer comprising a sequence of SEQ ID NO:7 or a sequence having at least 80%, preferably at least 85%, more preferably at least 90%, more preferably at least 95% sequence identity therewith, and a reverse primer comprising a sequence of SEQ ID NO:8 or a sequence having at least 80%, preferably at least 85%, more preferably at least 90%, more preferably at least 95% sequence identity therewith,
   d. Primer pair 4 consisting of a forward primer comprising a sequence of SEQ ID NO:10 or a sequence having at least 80%, preferably at least 85%, more preferably at least 90%, more preferably at least 95% sequence identity therewith, and a reverse primer comprising a sequence of SEQ ID NO:11 or a sequence having at least 80%, preferably at least 85%, more preferably at least 90%, more preferably at least 95% sequence identity therewith,
   e. Primer pair 5 consisting of a forward primer comprising a sequence of SEQ ID NO: 13 or a sequence having at least 80%, preferably at least 85%, more preferably at least 90%, more preferably at least 95% sequence identity therewith, and a reverse primer comprising a sequence of SEQ ID NO:14 or a sequence having at least 80%, preferably at least 85%, more preferably at least 90%, more preferably at least 95% sequence identity therewith,
   f. Primer pair 6 consisting of a forward primer comprising a sequence of SEQ ID NO: 16 or a sequence having at least 80%, preferably at least 85%, more preferably at least 90%, more preferably at least 95% sequence identity therewith, and a reverse primer comprising a sequence of SEQ ID NO:17 or a sequence having at least 80%, preferably at least 85%, more preferably at least 90%, more preferably at least 95% sequence identity therewith,
   g. Primer pair 7 consisting of a forward primer comprising a sequence of SEQ ID NO: 19 or a sequence having at least 80%, preferably at least 85%, more preferably at least 90%, more preferably at least 95% sequence identity therewith, and a reverse primer comprising a sequence of SEQ ID NO:20 or a sequence having at least 80%, preferably at least 85%, more preferably at least 90%, more preferably at least 95% sequence identity therewith, and
   h. Primer pair 8 consisting of a forward primer comprising a sequence of SEQ ID NO:22 or a sequence having at least 80%, preferably at least 85%, more preferably at least 90%, more preferably at least 95% sequence identity therewith, and a reverse primer comprising a sequence of SEQ ID NO:23 or a sequence having at least 80%, preferably at least 85%, more preferably at least 90%, more preferably at least 95% sequence identity therewith.

All primers of the primer pairs indicated under points a. to h. above may comprise a sequence having at least 80%, more preferably at least 85%, more preferably at least 90%, more preferably at least 95% sequence identity with the sequences of the indicated SEQ ID NOs.

According to the present invention, the PCR reaction of each primer pair may be performed in a separate tube. Thus, 8 separate PCR reactions may be performed. However, it is preferable that the number of separate PCR reactions is reduced in order to save time and resources. Preferably, all PCR reactions are performed in a single tube.

In preferred embodiments of the present invention, the forward and reverse primers do not only comprise the sequences of SEQ ID NOs: 1, 2, 4, 5, 7, 8, 10, 11, 13, 14, 16, 17, 19, 20, 22, 23 as indicated above or variants having a sequence identity of at least 80%, more preferably at least 85% more preferably at least 90%, more preferably at least 95% therewith but even consist of these sequences.

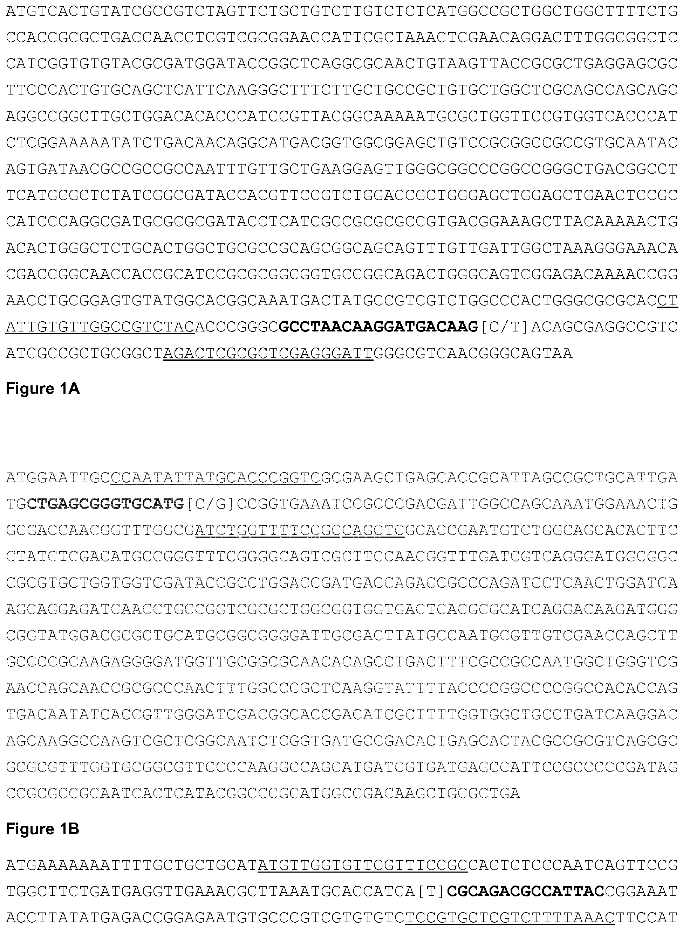

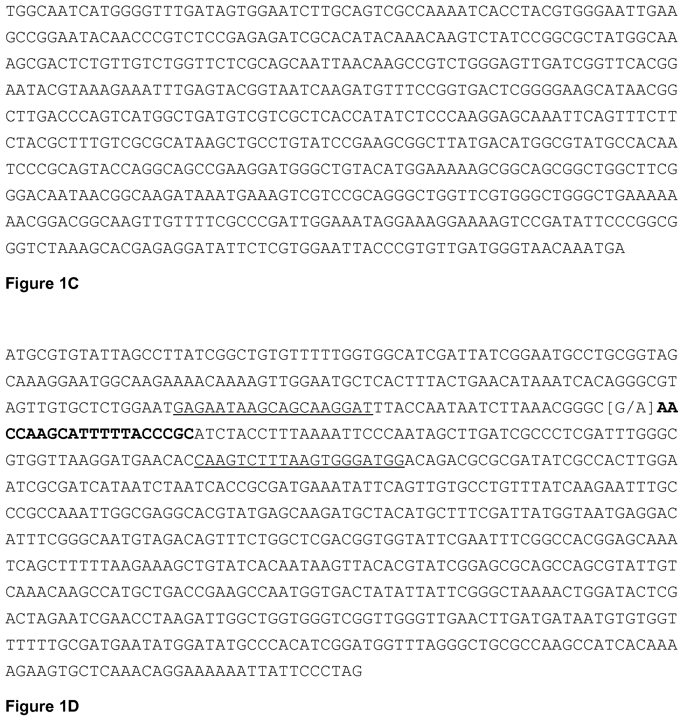

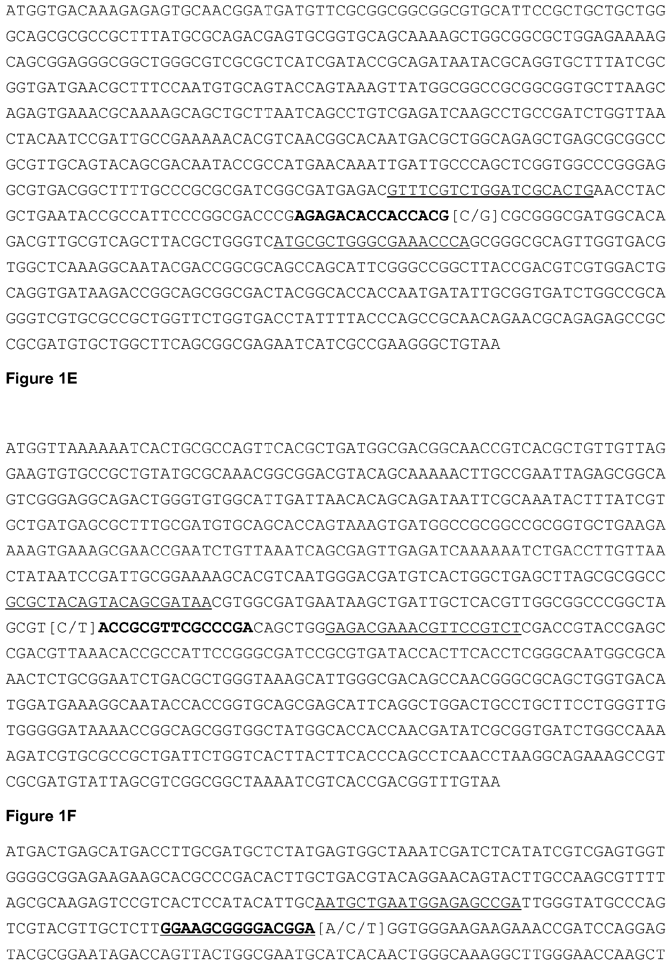

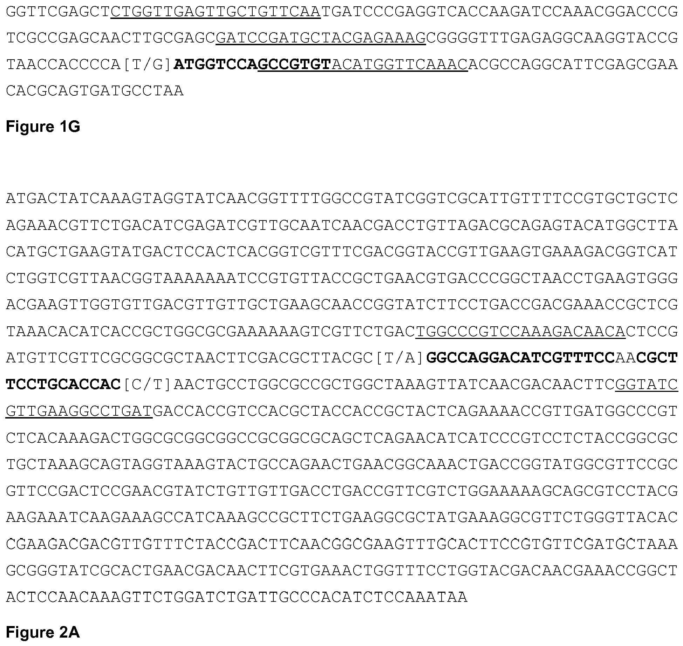

In the methods described above, the step of performing PCR reactions comprising primer pairs 1 to 8 preferably results in PCR products, in particular in amplification of regions of interest comprised in the KPC-2 coding sequence (SEQ ID NO:87), the NDM-1 coding sequence (SEQ ID NO:88), the OXA-9 coding sequence (SEQ ID NO:89), the OXA-48 coding sequence (SEQ ID NO:90), the CTX-M-9 coding sequence (SEQ ID NO:91), the CTX-M-15 coding sequence (SEQ ID NO:92), and the AAC(6')-lb-cr coding sequence (SEQ ID NO:93), or in variants or homologous coding sequences having at least 80%, more preferably at least 90%, more preferably at least 95%, more preferably at least 98%, even more preferably at least 99% sequence identity with the indicated coding sequences in case the respective coding sequences are present in the analyzed sample. Exemplary binding regions of the amplification primers and of the SNPs of interest are shown in figure 1.

Particularly preferably, primer pair 1 results in amplification of a region comprising the nucleotide at position 814 of the KPC-2 coding sequence (SEQ ID NO:87), primer pair 2 results in amplification of a region comprising the nucleotide at position 82 of the NDM-1 coding sequence (SEQ ID NO:88), primer pair 3 results in amplification of a region comprising the nucleotide at position 104 of the OXA-9 coding sequence (SEQ ID NO:89), primer pair 4 results in amplification of a region comprising the nucleotide at position 186 of the OXA-48 coding sequence (SEQ ID NO:90), primer pair 5 results in amplification of a region comprising the nucleotide at position 556 of the CTX-M-9 coding sequence (SEQ ID NO:91), primer pair 6 results in amplification of a region comprising the nucleotide at position 453 of the CTX-M-15 coding sequence (SEQ ID NO:92), primer pair 7 results in amplification of a region comprising the nucleotide at position 223 of the AAC(6')-lb-cr coding sequence (SEQ ID NO:93) and primer pair 8 results in amplification of a region comprising the nucleotide at position 454 of the AAC(6')-lb-cr coding sequence (SEQ ID NO:93), or at analogous positions in variants or homologous coding sequences having at least 80%, more preferably at least 90%, more preferably at least 95%, more preferably at least 98%, even more preferably at least 99% sequence identity with the indicated coding sequences.

In addition to the step A) of performing PCR reactions comprising the indicated primer pairs, the methods of the present invention preferably comprise the additional step of:
B) Allowing extension primers to bind to the PCR products obtained from the PCR reactions of step A), wherein preferably at least one, more preferably exactly one extension primer is binding to each of the PCR products obtained from step A) of the method.

Thus, preferably an extension primer is allowed to bind to the PCR product of primer pair 1, another extension primer is allowed to bind to the PCR product of primer pair 2, another extension primer is allowed to bind to the PCR product of primer pair 3, another extension primer is allowed to bind to the PCR product of primer pair 4, another extension primer is allowed to bind to the PCR product of primer pair 5, another extension primer is allowed to bind to the PCR product of primer pair 6, another extension primer is allowed to bind to the PCR product of primer pair 7 and another extension primer is allowed to bind to the PCR product of primer pair 8. Exemplary binding regions of extension primers are illustrated in figure 1.

Sequences that are preferably comprised by these extension primers are presented in the sequence listing of the present invention and are summarized in the following table 4. The sequences indicated in table 4 are most preferred. However, variants having a sequence identity of at least 80%, more preferably at least 85% more preferably at least 90%, more preferably at least 93% with the sequences indicated in table 4 are also preferable sequences of the present invention.

**Table 4**

| **Primer name** | **SEQ ID NO:** | **Comprised sequence (5'-3')** |
|---|---|---|
| KPC-2 extension | 3 | GCCTAACAAGGATGACAAG |
| NDM-1 extension | 50 | CTGAGCGGGTGCATG |
| OXA-9 extension | 53 | GTAATGGCGTCTGCG |
| OXA-48 extension | 56 | GCGGGTAAAAATGCTTGGTT |
| CTX-M-9 extension | 59 | AGAGACACCACCACG |
| CTX-M-15 extension | 18 | TCGGGCGAACGCGGT |
| AAC(6')-lb-cr (223) extension | 64 | GGAAGCGGGGACGGA |
| AAC(6')-lb-cr (454) extension | 24 | ACACGGCTGGACCAT |

Preferably, the following the following extension primers are allowed to bind to the PCR products obtained from the PCR reactions of step A):
a. KPC-2 extension primer comprising a sequence of SEQ ID NO:3 or a sequence having a sequence identity of at least 80%, more preferably at least 85% more preferably at least 90%, more preferably at least 93% with SEQ ID NO:3,
b. NDM-1 extension primer comprising a sequence of SEQ ID NO:50 or a sequence having a sequence identity of at least 80%, more preferably at least 85% more preferably at least 90%, more preferably at least 93% with SEQ ID NO:50,
c. OXA-9 extension primer comprising a sequence of SEQ ID NO:53 or a sequence having a sequence identity of at least 80%, more preferably at least 85% more preferably at least 90%, more preferably at least 93% with SEQ ID NO:53,
d. OXA-48 extension primer comprising a sequence of SEQ ID NO:56 or a sequence having a sequence identity of at least 80%, more preferably at least 85% more preferably at least 90%, more preferably at least 93% with SEQ ID NO:56,
e. CTX-M-9 extension primer comprising a sequence of SEQ ID NO:59 or a sequence having a sequence identity of at least 80%, more preferably at least 85% more preferably at least 90%, more preferably at least 93% with SEQ ID NO:59,
f. CTX-M-15 extension primer comprising a sequence of SEQ ID NO:18 or a sequence having a sequence identity of at least 80%, more preferably at least 85% more preferably at least 90%, more preferably at least 93% with SEQ ID NO:18,
g. AAC(6')-lb-cr (223) extension primer comprising a sequence of SEQ ID NO:64 or a sequence having a sequence identity of at least 80%, more preferably at least 85% more preferably at least 90%, more preferably at least 93% with SEQ ID NO:64, and
h. AAC(6')-lb-cr (454) extension primer comprising a sequence of SEQ ID NO:24 or a sequence having a sequence identity of at least 80%, more preferably at least 85% more preferably at least 90%, more preferably at least 93% with SEQ ID NO:24.

All primers indicated under points a. to h. above may comprise a sequence having at least 80%, more preferably at least 85%, more preferably at least 90%, more preferably at least 93% sequence identity with the sequences of the indicated SEQ ID NOs.

In embodiments of the present invention, the extension primers do not only comprise the sequences indicated in table 4 or variants having a sequence identity of at least 80%, more preferably at least 85% more preferably at least 90%, more preferably at least 93% therewith but even consist of these sequences. However, the present inventors found that it may be advantageous if non-complementary bases are added at the 5'-end of the primer so that each primer has a detectable different mass. Preferably, in addition to the sequences described in table 4 or to variants thereof as indicated above, the extension primers comprise at least 1, more preferably at least 2 non-complementary bases at the 5'-end. However, the number of non-complementary bases at the 5'-end should be limited. Preferably, the extension primers comprise at most 10, more preferably at most 5, more preferably at most 4 non-complementary bases at the 5'-end. It was found that non-complementary bases at the 5'-end are particularly preferred for NDM-1 extension primers, OXA-9 extension primers, OXA-48 extension primers, CTX-M-9 extension primers and AAC(6')-lb-cr (223) extension primers.

Sequences that are comprised in most preferred extension primers of the present invention comprising non-complementary bases at the 5'-end are summarized in the following table 5. However, variants having a sequence identity of at least 80%, more preferably at least 85% more preferably at least 90%, more preferably at least 93% with the sequences indicated in table 5 are also preferable sequences of the present invention.

**Table 5**

| **Primer name** | **SEQ ID NO:** | **Comprised sequence (5'-3')** |
|---|---|---|
| NDM-1 extension long | 6 | GGGACTGAGCGGGTGCATG |
| OXA-9 extension long | 9 | CTGTAATGGCGTCTGCG |
| OXA-48 extension long | 12 | GGGCGGGTAAAAATGCTTGGTT |
| CTX-M-9 extension long | 15 | TTAGAGACACCACCACG |
| AAC(6')-lb-cr (223) extension long | 21 | CGGAAGCGGGGACGGA |

Preferably, the following extension primers are allowed to bind to the PCR products obtained from the PCR reactions of step A):
a. KPC-2 extension primer comprising a sequence of SEQ ID NO:3 or a sequence having a sequence identity of at least 80%, more preferably at least 85% more preferably at least 90%, more preferably at least 93% with SEQ ID NO:3,
b. NDM-1 extension primer comprising a sequence of SEQ ID NO:6 or a sequence having a sequence identity of at least 80%, more preferably at least 85% more preferably at least 90%, more preferably at least 93% with SEQ ID NO:6,
c. OXA-9 extension primer comprising a sequence of SEQ ID NO:9 or a sequence having a sequence identity of at least 80%, more preferably at least 85% more preferably at least 90%, more preferably at least 93% with SEQ ID NO:9,
d. OXA-48 extension primer comprising a sequence of SEQ ID NO:12 or a sequence having a sequence identity of at least 80%, more preferably at least 85% more preferably at least 90%, more preferably at least 93% with SEQ ID NO:12,
e. CTX-M-9 extension primer comprising a sequence of SEQ ID NO:15 or a sequence having a sequence identity of at least 80%, more preferably at least 85% more preferably at least 90%, more preferably at least 93% with SEQ ID NO:15,
f. CTX-M-15 extension primer comprising a sequence of SEQ ID NO:18 or a sequence having a sequence identity of at least 80%, more preferably at least 85% more preferably at least 90%, more preferably at least 93% with SEQ ID NO:18,
g. AAC(6')-lb-cr (223) extension primer comprising a sequence of SEQ ID NO:21 or a sequence having a sequence identity of at least 80%, more preferably at least 85% more preferably at least 90%, more preferably at least 93% with SEQ ID NO:21, and
h. AAC(6')-lb-cr (454) extension primer comprising a sequence of SEQ ID NO:24 or a sequence having a sequence identity of at least 80%, more preferably at least 85% more preferably at least 90%, more preferably at least 93% with SEQ ID NO:24.

All primers indicated under points a. to h. above may comprise a sequence having at least 80%, more preferably at least 85%, more preferably at least 90%, more preferably at least 93% sequence identity with the sequences of the indicated SEQ ID NOs.

Preferably, the extension primers indicated in table 5 do not only comprise the sequences indicated in table 5 or variants having a sequence identity of at least 80%, more preferably at least 85% more preferably at least 90%, more preferably at least 93% therewith but even consist of these sequences.

In addition to step A) of performing PCR reactions comprising the indicated primer pairs and step B) of allowing extension primers to bind to the PCR products obtained from step A), the methods of the present invention preferably comprise the additional step of:
C) Elongating the extension primers at the 3'-end by at least one nucleotide, preferably by exactly one nucleotide.

Preferably, the elongation of all extension primers is performed in a single tube, more preferably in the same tube, in which the PCR reactions have been performed.

Preferably, the methods of the present invention comprise the additional step of:
D) Identifying the at least one nucleotide, preferably the exactly one nucleotide that has been added to the extension primer.

Preferably, the nucleotide is determined by mass spectrometry. Thus, the masses of the elongated extension primers are preferably determined by mass spectrometry. This enables identification of the nucleotide that has been added to the extension primer by comparison of the added mass with the known masses of the different nucleotides.

Preferably, the methods of the present invention comprise the additional step of:
E) Determining information on both the phylogenetic origin and antibiotic resistances of the bacterial strain based on a comparison of the pattern of the determined nucleotides with respective predetermined patterns of bacterial strains, which phylogenetic origin and antibiotic resistances are known.

The PCR reactions of the methods of the present invention described above may additionally comprise further primer pairs. These additional primer pairs may result in amplification of further regions of interest of resistance conferring genes and/or in amplification of regions of interest of genes that do not confer resistance, in particular of house-keeping genes. The present inventors found that the identification of the phylogenetic origin can be particularly preferably improved if the results obtained from analysis of the coding sequences of the antibiotic resistance conferring genes (SEQ ID NOs: 87-93) are combined with phylogenetic information obtained by analysis of species-specific phylogeny markers, in particular of house-keeping genes. This is shown exemplarily in the present description for *Klebsiella pneumoniae*.

The present inventors have found that determination of information on the phylogenetic origin by the method of the present invention can be further improved if PCR reactions are performed that comprise further primer pairs in addition to primer pairs 1 to 8 described above. Sequences that are preferably comprised by these additional PCR primers are presented in the sequence listing of the present invention and are summarized in the following table 6. The sequences indicated in table 6 are most preferred. However, variants having a sequence identity of at least 80%, more preferably at least 85% more preferably at least 90%, more preferably at least 94% with the sequences indicated in table 6 are also preferable sequences of the present invention.

**Table 6**

| **Primer name** | **SEQ ID NO:** | **Comprised sequence (5'-3')** |
|---|---|---|
| gapA forward | 67 | TGGCCCGTCCAAAGACAACA |
| gapA reverse | 68 | ATCAGGCCTTCAACGATACC |
| infB (1608) forward | 73 | TGAAGAACGAACTGTCCCAG |
| infB (1608) reverse | 74 | GTCCAGCAGGTCGTCGATG |
| mdh (633) forward | 76 | GGGTCACCATTCTGCCTTTA |
| mdh (633) reverse | 77 | CGCCCGCTTTCGCTTCCAC |
| phoE (732) forward | 79 | ACAATATCTACCTGGCGACC |
| phoE (732) reverse | 80 | GTTGAAGTAGTAGGTCAGGC |
| phoE (867) forward | 82 | TCCCTCGGCTATGTGCTGT |
| phoE (867) reverse | 83 | CGAAGGCGTTCATGTTTTTG |
| rpoB (1819) forward | 84 | TGATTAACTCCCTGTCCGTG |
| rpoB (1819) reverse | 85 | CCTTCTTCGATAGCAGACAG |

By using a combination of corresponding forward and reverse primers, DNA fragments containing the SNP positions of the present invention can be excellently amplified, in particular as PCR products.

In embodiments of the present invention, the amplifying primers do not only comprise the sequences indicated in table 6 or variants having a sequence identity of at least 80%, more preferably at least 85% more preferably at least 90%, more preferably at least 94% therewith but even consist of these sequences. However, the present inventors found that the amplification is advantageously balanced if the amplification primers additionally comprise nucleotides at the 5'-end. Preferably, in addition to the sequences described in table 6 or to variants thereof as indicated above, the amplification primers comprise at least 5, more preferably at least 8, more preferably at least 9, more preferably at least 10 and even more preferably exactly 10 additional nucleotides at the 5'-end. The number of additional nucleotides at the 5'-end should be limited and therefore the amplification primers preferably comprise at most 20, more preferably at most 15, more preferably at most 12, more preferably at most 11 and even more preferably at most 10 additional nucleotides at the 5'-end. Sequences that are comprised in most preferred amplification primers of the present invention are summarized in the following table 7. However, variants having a sequence identity of at least 80%, more preferably at least 85% more preferably at least 90%, more preferably at least 95% with the sequences indicated in table 7 are also preferable sequences of the present invention.

**Table 7**

| **Primer name** | **SEQ ID NO:** | **Comprised sequence (5'-3')** |
|---|---|---|
| gapA long forward | 25 | ACGTTGGATGTGGCCCGTCCAAAGACAACA |
| gapA long reverse | 26 | ACGTTGGATGATCAGGCCTTCAACGATACC |
| infB (1608) long forward | 31 | ACGTTGGATGTGAAGAACGAACTGTCCCAG |
| infB (1608) long reverse | 32 | ACGTTGGATGGTCCAGCAGGTCGTCGATG |
| mdh (633) long forward | 34 | ACGTTGGATGGGGTCACCATTCTGCCTTTA |
| mdh (633) long reverse | 35 | ACGTTGGATGCGCCCGCTTTCGCTTCCAC |
| phoE (732) long forward | 37 | ACGTTGGATGACAATATCTACCTGGCGACC |
| phoE (732) long reverse | 38 | ACGTTGGATGGTTGAAGTAGTAGGTCAGGC |
| phoE (867) long forward | 40 | ACGTTGGATGTCCCTCGGCTATGTGCTGT |
| phoE (867) long reverse | 41 | ACGTTGGATGCGAAGGCGTTCATGTTTTTG |
| rpoB (1819) long forward | 43 | ACGTTGGATGTGATTAACTCCCTGTCCGTG |
| rpoB (1819) long reverse | 44 | ACGTTGGATGCCTTCTTCGATAGCAGACAG |

Preferably, the amplifying primers do not only comprise the sequences indicated in table 7 or variants having a sequence identity of at least 80%, more preferably at least 85% more preferably at least 90%, more preferably at least 95% therewith but even consist of these sequences.

Preferably, the PCR reactions of step A) of the methods of the present invention described above for determining in a bacterial sample information on both the phylogenetic origin and the antibiotic resistances of bacterial strains additionally comprise at least one, more preferably at least two, more preferably at least three, more preferably at least four, more preferably at least five, more preferably all six of the following primer pairs:
i. Primer pair 9 consisting of a forward primer comprising a sequence of SEQ ID NO:67 or a sequence having at least 80%, preferably at least 85%, more preferably at least 90%, more preferably at least 94% sequence identity therewith, and a reverse primer comprising a sequence of SEQ ID NO:68 or a sequence having at least 80%, preferably at least 85%, more preferably at least 90%, more preferably at least 94% sequence identity therewith,
j. Primer pair 10 consisting of a forward primer comprising a sequence of SEQ ID NO:73 or a sequence having at least 80%, preferably at least 85%, more preferably at least 90%, more preferably at least 94% sequence identity therewith, and a reverse primer comprising a sequence of SEQ ID NO:74 or a sequence having at least 80%, preferably at least 85%, more preferably at least 90%, more preferably at least 94% sequence identity therewith,
k. Primer pair 11 consisting of a forward primer comprising a sequence of SEQ ID NO:76 or a sequence having at least 80%, preferably at least 85%, more preferably at least 90%, more preferably at least 94% sequence identity therewith, and a reverse primer comprising a sequence of SEQ ID NO:77 or a sequence having at least 80%, preferably at least 85%, more preferably at least 90%, more preferably at least 94% sequence identity therewith,
l. Primer pair 12 consisting of a forward primer comprising a sequence of SEQ ID NO:79 or a sequence having at least 80%, preferably at least 85%, more preferably at least 90%, more preferably at least 94% sequence identity therewith, and a reverse primer comprising a sequence of SEQ ID NO:80 or a sequence having at least 80%, preferably at least 85%, more preferably at least 90%, more preferably at least 94% sequence identity therewith,
m. Primer pair 13 consisting of a forward primer comprising a sequence of SEQ ID NO:82 or a sequence having at least 80%, preferably at least 85%, more preferably at least 90%, more preferably at least 94% sequence identity therewith, and a reverse primer comprising a sequence of SEQ ID NO:83 or a sequence having at least 80%, preferably at least 85%, more preferably at least 90%, more preferably at least 94% sequence identity therewith, and
n. Primer pair 14 consisting of a forward primer comprising a sequence of SEQ ID NO:84 or a sequence having at least 80%, preferably at least 85%, more preferably at least 90%, more preferably at least 94% sequence identity therewith, and a reverse primer comprising a sequence of SEQ ID NO:85 or a sequence having at least 80%, preferably at least 85%, more preferably at least 90%, more preferably at least 94% sequence identity therewith.

All primers of the primer pairs indicated under points i. to n. above may comprise a sequence having at least 80%, more preferably at least 85%, more preferably at least 90%, more preferably at least 94% sequence identity with the sequences of the indicated SEQ ID NOs.

According to the present invention, the PCR reaction of each primer pair may be performed in a separate tube. Thus, for example, 14 separate PCR reactions may be performed with primer pairs 1 to 14 described above. However, it is preferable that the number of separate PCR reactions is reduced in order to save time and resources. Preferably, all PCR reactions are performed in a single tube.

In embodiments of the present invention, the forward and reverse primers of primer pairs 9 to 14 do not only comprise the sequences of SEQ ID NOs: 67, 68, 73, 74, 76, 77, 79, 80, 82-85 as indicated above or variants having a sequence identity of at least 80%, more preferably at least 85% more preferably at least 90%, more preferably at least 94% therewith but even consist of these sequences. However, the present inventors found that the amplification is advantageously balanced if the forward and reverse primers additionally comprise nucleotides at the 5'-end. Furthermore, this increases the masses of unused primers (out of the mass range on the spectra).

Therefore, the PCR reactions of step A) of the methods of the present invention described above for determining in a bacterial sample information on both the phylogenetic origin and the antibiotic resistances of bacterial strains additionally comprise at least one, more preferably at least two, more preferably at least three, more preferably at least four, more preferably at least five, more preferably all six of the following primer pairs:
i. Primer pair 9 consisting of a forward primer comprising a sequence of SEQ ID NO:25 or a sequence having at least 80%, preferably at least 85%, more preferably at least 90%, more preferably at least 95% sequence identity therewith, and a reverse primer comprising a sequence of SEQ ID NO:26 or a sequence having at least 80%, preferably at least 85%, more preferably at least 90%, more preferably at least 95% sequence identity therewith,
j. Primer pair 10 consisting of a forward primer comprising a sequence of SEQ ID NO:31 or a sequence having at least 80%, preferably at least 85%, more preferably at least 90%, more preferably at least 95% sequence identity therewith, and a reverse primer comprising a sequence of SEQ ID NO:32 or a sequence having at least 80%, preferably at least 85%, more preferably at least 90%, more preferably at least 95% sequence identity therewith,
k. Primer pair 11 consisting of a forward primer comprising a sequence of SEQ ID NO:34 or a sequence having at least 80%, preferably at least 85%, more preferably at least 90%, more preferably at least 95% sequence identity therewith, and a reverse primer comprising a sequence of SEQ ID NO:35 or a sequence having at least 80%, preferably at least 85%, more preferably at least 90%, more preferably at least 95% sequence identity therewith,
l. Primer pair 12 consisting of a forward primer comprising a sequence of SEQ ID NO:37 or a sequence having at least 80%, preferably at least 85%, more preferably at least 90%, more preferably at least 95% sequence identity therewith, and a reverse primer comprising a sequence of SEQ ID NO:38 or a sequence having at least 80%, preferably at least 85%, more preferably at least 90%, more preferably at least 95% sequence identity therewith,
m. Primer pair 13 consisting of a forward primer comprising a sequence of SEQ ID NO:40 or a sequence having at least 80%, preferably at least 85%, more preferably at least 90%, more preferably at least 95% sequence identity therewith, and a reverse primer comprising a sequence of SEQ ID NO:41 or a sequence having at least 80%, preferably at least 85%, more preferably at least 90%, more preferably at least 95% sequence identity therewith, and
n. Primer pair 14 consisting of a forward primer comprising a sequence of SEQ ID NO:43 or a sequence having at least 80%, preferably at least 85%, more preferably at least 90%, more preferably at least 95% sequence identity therewith, and a reverse primer comprising a sequence of SEQ ID NO:44 or a sequence having at least 80%, preferably at least 85%, more preferably at least 90%, more preferably at least 95% sequence identity therewith.

All primers of the primer pairs indicated under points i. to n. above may comprise a sequence having at least 80%, more preferably at least 85%, more preferably at least 90%, more preferably at least 95% sequence identity with the sequences of the indicated SEQ ID NOs.

According to the present invention, the PCR reaction of each primer pair may be performed in a separate tube. Thus, for example, 14 separate PCR reactions may be performed with primer pairs 1 to 14 described above. However, it is preferable that the number of separate PCR reactions is reduced in order to save time and resources. Preferably, all PCR reactions are performed in a single tube.

In preferred embodiments of the present invention, the forward and reverse primers do not only comprise the sequences of SEQ ID NOs: 25, 26, 31, 32, 34, 35, 37, 38, 40, 41, 43, 44 as indicated above or variants having a sequence identity of at least 80%, more preferably at least 85% more preferably at least 90%, more preferably at least 95% therewith but even consist of these sequences.

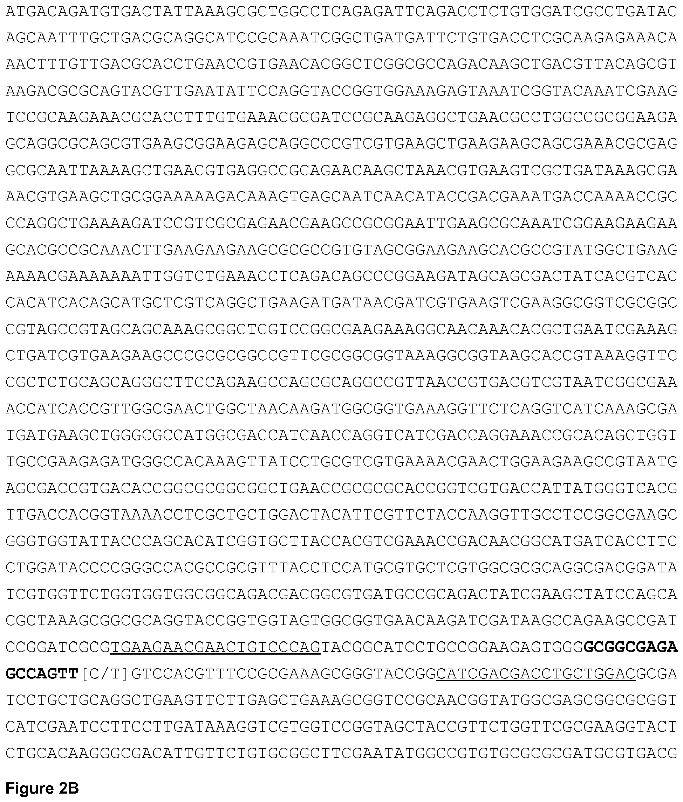

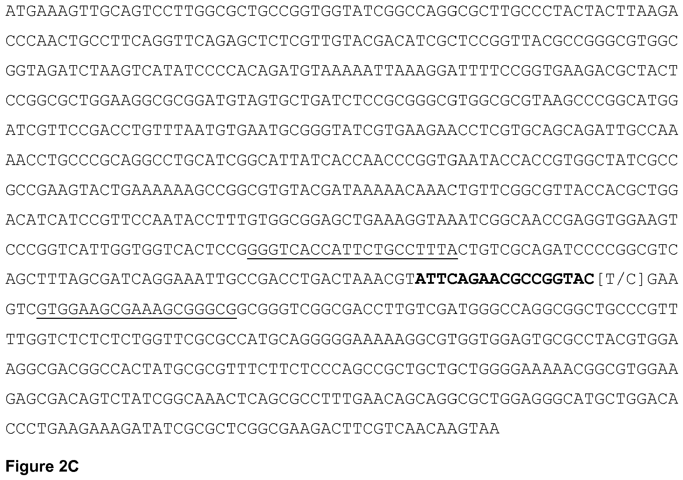

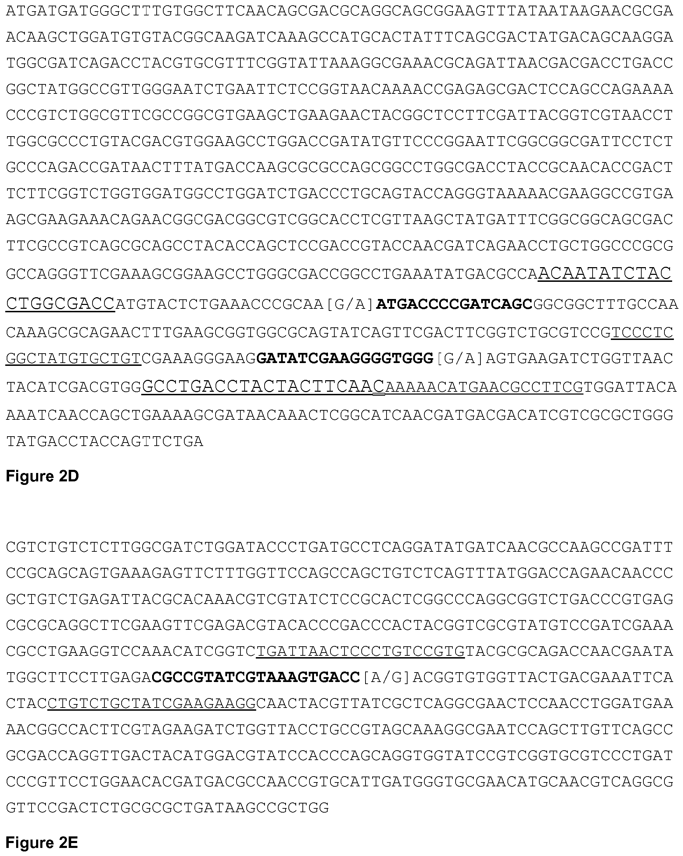

Preferably, primer pair 9 results in amplification of a region comprising the nucleotide at position 420 and at position 456 of the gapA coding sequence (SEQ ID NO:28), primer pair 10 results in amplification of a region comprising the nucleotide at position 1608 of the infB coding sequence (SEQ ID NO:29), primer pair 11 results in amplification of a region comprising the nucleotide at position 633 of the mdh coding sequence (SEQ ID NO:70), primer pair 12 results in amplification of a region comprising the nucleotide at position 732 of the phoE coding sequence (SEQ ID NO:71), primer pair 13 results in amplification of a region comprising the nucleotide at position 867 of the phoE coding sequence (SEQ ID NO:71) and primer pair 14 results in amplification of a region comprising the nucleotide at position 1819 of the rpoB coding sequence (SEQ ID NO:94), or at analogous positions in variants or homologous coding sequences having at least 80%, more preferably at least 90%, more preferably at least 95%, more preferably at least 98%, even more preferably at least 99% sequence identity with the indicated coding sequences. Exemplary binding regions of the amplifications primers and of the SNPs of interest are shown in figure 2.

In step B) of the methods of the present invention, extension primers are preferably allowed to bind to the PCR products obtained from the PCR reactions of primer pairs 9 to 14, wherein preferably at least one, more preferably exactly one extension primer is binding to each of the obtained PCR products. Particularly preferred, exactly one extension primer is binding to the PCR products obtained from the PCR reactions of primer pairs 10 to 14 and exactly two extension primers are binding to the PCR products obtained from the PCR reactions of primer pair 9. Exemplary binding regions of extension primers are shown in figure 2.

Sequences that are preferably comprised by these extension primers are presented in the sequence listing of the present invention and are summarized in the following table 8. The sequences indicated in table 8 are most preferred. However, variants having a sequence identity of at least 80%, more preferably at least 85% more preferably at least 90%, more preferably at least 93% with the sequences indicated in table 8 are also preferable sequences of the present invention. The letter "I" in the sequences in table 8 indicates inosine.

**Table 8**

| **Primer name** | **SEQ ID NO:** | **Comprised sequence (5'-3')** |
|---|---|---|
| gapA (420) extension | 69 | GGAAACGATGTCCTGGCC |
| gapA (456) extension | 72 | CGCITCCTGCACCAC |
| infB (1608) extension | 75 | GCGGCGAGAGCCAGTT |
| mdh (633) extension | 78 | ATTCAIAAIGCCGGTAC |
| phoE (732) extension | 81 | GCTGATCGGGGTCAT |
| phoE (867) extension | 42 | GATATIGAAGGGGTGGG |
| rpoB (1819) extension | 86 | CICCGTATCGTAAAGTGACC |

Preferably, at least one, more preferably at least two, more preferably at least three, more preferably at least four, more preferably at least five, more preferably at least six, more preferably all seven of the following extension primers are allowed to bind to the PCR products obtained from the PCR reactions of step A) in addition to the extension primers of the antibiotic resistance markers as indicated above:
i. gapA (420) extension primer comprising a sequence of SEQ ID NO:69 or a sequence having a sequence identity of at least 80%, more preferably at least 85% more preferably at least 90%, more preferably at least 93% with SEQ ID NO:69,
j. gapA (456) extension primer comprising a sequence of SEQ ID NO:72 or a sequence having a sequence identity of at least 80%, more preferably at least 85% more preferably at least 90%, more preferably at least 93% with SEQ ID NO:72,
k. infB (1608) extension primer comprising a sequence of SEQ ID NO:75 or a sequence having a sequence identity of at least 80%, more preferably at least 85% more preferably at least 90%, more preferably at least 93% with SEQ ID NO:75,
l. mdh (633) extension primer comprising a sequence of SEQ ID NO:78 or a sequence having a sequence identity of at least 80%, more preferably at least 85% more preferably at least 90%, more preferably at least 93% with SEQ ID NO:78,
m. phoE (732) extension primer comprising a sequence of SEQ ID NO:81 or a sequence having a sequence identity of at least 80%, more preferably at least 85% more preferably at least 90%, more preferably at least 93% with SEQ ID NO:81,
n. phoE (867) extension primer comprising a sequence of SEQ ID NO:42 or a sequence having a sequence identity of at least 80%, more preferably at least 85% more preferably at least 90%, more preferably at least 93% with SEQ ID NO:42,
o. rpoB (1819) extension primer comprising a sequence of SEQ ID NO:86 or a sequence having a sequence identity of at least 80%, more preferably at least 85% more preferably at least 90%, more preferably at least 93% with SEQ ID NO:86.

All primers indicated under points i. to o. above may comprise a sequence having at least 80%, more preferably at least 85%, more preferably at least 90%, more preferably at least 93% sequence identity with the sequences of the indicated SEQ ID NOs.

In embodiments of the present invention, the extension primers do not only comprise the sequences indicated in table 8 or variants having a sequence identity of at least 80%, more preferably at least 85% more preferably at least 90%, more preferably at least 93% therewith but even consist of these sequences. However, the present inventors found that it may be advantageous if non-complementary bases are added at the 5'-end of the primer so that each primer has a detectable different mass. Preferably, in addition to the sequences described in table 8 or to variants thereof as indicated above, the extension primers comprise at least 1, more preferably at least 2 non-complementary bases at the 5'-end. However, the number of non-complementary bases at the 5'-end should be limited. Preferably, the extension primers comprise at most 10, more preferably at most 5, more preferably at most 4 non-complementary bases at the 5'-end. It was found that non-complementary bases at the 5'-end are particularly preferred for gapA (420) extension primers, gapA (456) extension primers, infB (1608) extension primers, mdh (633) extension primers, phoE (732) extension primers and rpoB (1819) extension primers.

Sequences that are comprised in most preferred extension primers of the present invention comprising non-complementary bases at the 5'-end are summarized in the following table 9. However, variants having a sequence identity of at least 80%, more preferably at least 85% more preferably at least 90%, more preferably at least 93% with the sequences indicated in table 5 are also preferable sequences of the present invention. The letter "I" in the sequences in table 9 indicates inosine.

**Table 9**

| **Primer name** | **SEQ ID NO:** | **Comprised sequence (5'-3')** |
|---|---|---|
| gapA (420) extension long | 27 | GAGGGGAAACGATGTCCTGGCC |
| gapA (456) extension long | 30 | CCGCITCCTGCACCAC |
| infB (1608) extension long | 33 | GTGTGCGGCGAGAGCCAGTT |
| mdh (633) extension long | 36 | AAGTATTCAIAAIGCCGGTAC |
| phoE (732) extension long | 39 | GGCTGATCGGGGTCAT |
| rpoB (1819) extension long | 45 | GGCICCGTATCGTAAAGTGACC |

Preferably, at least one, more preferably at least two, more preferably at least three, more preferably at least four, more preferably at least five, more preferably at least six, more preferably all seven of the following extension primers are allowed to bind to the PCR products obtained from the PCR reactions of step A) in addition to the extension primers of the antibiotic resistance markers as indicated above:
i. gapA (420) extension primer comprising a sequence of SEQ ID NO:27 or a sequence having a sequence identity of at least 80%, more preferably at least 85% more preferably at least 90%, more preferably at least 93% with SEQ ID NO:27,
j. gapA (456) extension primer comprising a sequence of SEQ ID NO:30 or a sequence having a sequence identity of at least 80%, more preferably at least 85% more preferably at least 90%, more preferably at least 93% with SEQ ID NO:30,
k. infB (1608) extension primer comprising a sequence of SEQ ID NO:33 or a sequence having a sequence identity of at least 80%, more preferably at least 85% more preferably at least 90%, more preferably at least 93% with SEQ ID NO:33,
l. mdh (633) extension primer comprising a sequence of SEQ ID NO:36 or a sequence having a sequence identity of at least 80%, more preferably at least 85% more preferably at least 90%, more preferably at least 93% with SEQ ID NO:36,
m. phoE (732) extension primer comprising a sequence of SEQ ID NO:39 or a sequence having a sequence identity of at least 80%, more preferably at least 85% more preferably at least 90%, more preferably at least 93% with SEQ ID NO:39,
n. phoE (867) extension primer comprising a sequence of SEQ ID NO:42 or a sequence having a sequence identity of at least 80%, more preferably at least 85% more preferably at least 90%, more preferably at least 93% with SEQ ID NO:42,
o. rpoB (1819) extension primer comprising a sequence of SEQ ID NO:45 or a sequence having a sequence identity of at least 80%, more preferably at least 85% more preferably at least 90%, more preferably at least 93% with SEQ ID NO:45.

All primers indicated under points i. to o. above may comprise a sequence having at least 80%, more preferably at least 85%, more preferably at least 90%, more preferably at least 93% sequence identity with the sequences of the indicated SEQ ID NOs.

Preferably, the extension primers indicated in table 9 do not only comprise the sequences indicated in table 9 or variants having a sequence identity of at least 80%, more preferably at least 85% more preferably at least 90%, more preferably at least 93% therewith but even consist of these sequences.

In step C) of the methods of the present invention, the extension primers described above are preferably elongated at the 3'-end by at least one nucleotide, preferably by exactly one nucleotide. Preferably, the elongation of all extension primers is performed in a single tube, more preferably in the same tube, in which the PCR reactions have been performed.

In step D) of the methods of the present invention, the at least one nucleotide, preferably exactly one nucleotide that has been added to the extension primer is determined. Preferably, the nucleotide is determined by mass spectrometry. Thus, the masses of the elongated extension primers are preferably determined by mass spectrometry. This enables identification of the nucleotide that has been added to the extension primer by comparison of the added mass with the known masses of the different nucleotides.

In step E) of the methods of the present invention, information on the phylogenetic origin is preferably determined based on a comparison of the pattern of the determined nucleotides with respective predetermined patterns of bacterial strain, which phylogenetic origin is known. In particular, the present inventors have found that determination of information on the phylogenetic origin by the method of the present invention can be further improved if additional information on phylogenetic origin is generated from PCR reactions comprising at least one of primer pairs 9 to 14 in addition to primer pairs 1 to 8 described above.

Preferably, the primers of the present invention have length of at most 100 nucleotides, more preferably at most 50 nucleotides, more preferably at most 40 nucleotides.

In the methods of the present invention, the nucleotide being present at certain position may be determined by any known sequencing method such as Sanger sequencing (dideoxy chain-termination method), Maxam-Gilbert sequencing, shotgun sequencing and next-generation sequencing methods such as pyrosequencing. However, the present inventors found that specifically satisfying results with regard to costs as well as specificity and sensitivity of detection are obtained when the nucleotides being present at certain positions are determined by a method that comprises a step of detection by mass spectrometry. A preferred mass spectrometry based detection method is MALDI-TOF (Matrix-Assisted Laser Desorption/Ionization-Time Of Flight). In a preferred embodiment, determination of nucleotides involves PCR based DNA amplification and subsequent detection of the nucleotide by mass spectrometry.

In a particular preferred embodiment, determination of the nucleotides involves a process, which will in the following be termed "*MassArray*". In such MassArray, relevant regions of bacterial DNA containing SNPs of interest are amplified by PCR in a first step. Subsequently, an extension primer binding to the DNA of interest just downstream of the nucleotide to be determined is elongated by at least one nucleotide at its 3'-end. Such elongated extension primer is then analyzed by mass spectrometry in order to determine the nucleotide that has been added to the primer and which is complementary to the nucleotide of interest, thus allowing determination of the nucleotide of interest.

In preferred embodiments, the extension primer is elongated by exactly one nucleotide. This is preferably achieved by use of chain terminating nucleotides, thus by nucleotides that prevent further elongation of the extension primer once they have been added to the 3'-end of the primer. Chain terminating nucleotides of the present invention are for example dideoxynucleotides (ddNTPs). However, the mass differences between different ddNTPs, in particular between ddATP and ddTTP, are comparably small, which complicates distinguishing by mass spectrometry. Therefore, particularly preferred chain terminating nucleotides of the present invention are acyclonucleotides (acyNTPs). Acyclonucleotides are chain terminators that lack a ribose 3'-OH required for further extension. Once an acyclonucleotide is incorporated by a DNA polymerase, the substrate can no longer be further extended. Acyclonucleotides are especially useful in applications with archaeon DNA polymerases, more preferably with Therminator DNA Polymerase. Therminator DNA polymerase is an engineered enzyme with an increased capacity to incorporate analogs with altered sugars, such as ribonucleotides, dideoxynucleotides, 2' deoxynucleotides and especially acyclo-base analogs.

The present inventors found that MassArray is a preferred technology for determination of the nucleotide of interest, particularly in combination with multiplex PCR, because it is rapid and robust and allows for the detection of many nucleotides of interest at reasonable costs. In particular, the present inventors found that the extension primers described above are specifically suitable for detection of the nucleotides of interest by MassArray.

The methods of the present invention may comprise determination of nucleotides present at further gene positions. Determination of more nucleotides renders the method more laborious and complex. However, on the other hand, sensitivity of determination of information on phylogenetic origin and/or antibiotic resistances may be further enhanced by determination of more nucleotides.

As found by the present inventors, the pattern of determined nucleotides of the present invention is very specific for individual bacterial isolates. Thus, by the methods of the present invention information on the phylogenetic origin of a bacterial isolate can be determined in addition to the antibiotic resistances by comparison of the pattern of the determined nucleotides with respective predetermined patterns of bacterial strains, of which phylogenetic origin and antibiotic resistances are known.

The present invention also comprises a kit for performing the method of the invention, wherein the kit comprises
(i) sets of PCR primers for amplification of bacterial DNA regions that include the nucleotides to be determined, and
(ii) extension primers for determining which nucleotide is present
   a. at position 814 in the KPC-2 coding sequence (SEQ ID NO:87),
   b. at position 82 in the NDM-1 coding sequence (SEQ ID NO:88),
   c. at position 104 in the OXA-9 coding sequence (SEQ ID NO:89),
   d. at position 186 in the OXA-48 coding sequence (SEQ ID NO:90),
   e. at position 556 in the CTX-M-9 coding sequence (SEQ ID NO:91),
   f. at position 453 in the CTX-M-15 coding sequence (SEQ ID NO:92),
   g. at position 223 in the AAC(6')-lb-cr coding sequence (SEQ ID NO:93), and
   h. at position 454 in the AAC(6')-lb-cr coding sequence (SEQ ID NO:93),
   or at analogous positions in variants or homologous coding sequences having at least 80% sequence identity with the indicated coding sequences.

Preferably, the variants or homologous coding sequences have a sequence identity with the coding sequences as depicted in SEQ ID NOs: 87-93 of at least 90%, more preferably of at least 95%, more preferably of at least 98%, even more preferably of at least 99%.

The present invention also relates to a kit comprising
(i) the following amplification primers:
   a. A forward primer comprising a sequence of SEQ ID NO:46 or a sequence having at least 80%, preferably at least 85%, more preferably at least 90%, more preferably at least 94% sequence identity therewith, and a reverse primer comprising a sequence of SEQ ID NO:47 or a sequence having at least 80%, preferably at least 85%, more preferably at least 90%, more preferably at least 94% sequence identity therewith,
   b. A forward primer comprising a sequence of SEQ ID NO:48 or a sequence having at least 80%, preferably at least 85%, more preferably at least 90%, more preferably at least 94% sequence identity therewith, and a reverse primer comprising a sequence of SEQ ID NO:49 or a sequence having at least 80%, preferably at least 85%, more preferably at least 90%, more preferably at least 94% sequence identity therewith,
   c. A forward primer comprising a sequence of SEQ ID NO:51 or a sequence having at least 80%, preferably at least 85%, more preferably at least 90%, more preferably at least 94% sequence identity therewith, and a reverse primer comprising a sequence of SEQ ID NO:52 or a sequence having at least 80%, preferably at least 85%, more preferably at least 90%, more preferably at least 94% sequence identity therewith,
   d. A forward primer comprising a sequence of SEQ ID NO:54 or a sequence having at least 80%, preferably at least 85%, more preferably at least 90%, more preferably at least 94% sequence identity therewith, and a reverse primer comprising a sequence of SEQ ID NO:55 or a sequence having at least 80%, preferably at least 85%, more preferably at least 90%, more preferably at least 94% sequence identity therewith,
   e. A forward primer comprising a sequence of SEQ ID NO:57 or a sequence having at least 80%, preferably at least 85%, more preferably at least 90%, more preferably at least 94% sequence identity therewith, and a reverse primer comprising a sequence of SEQ ID NO:58 or a sequence having at least 80%, preferably at least 85%, more preferably at least 90%, more preferably at least 94% sequence identity therewith,
   f. A forward primer comprising a sequence of SEQ ID NO:60 or a sequence having at least 80%, preferably at least 85%, more preferably at least 90%, more preferably at least 94% sequence identity therewith, and a reverse primer comprising a sequence of SEQ ID NO:61 or a sequence having at least 80%, preferably at least 85%, more preferably at least 90%, more preferably at least 94% sequence identity therewith,
   g. A forward primer comprising a sequence of SEQ ID NO:62 or a sequence having at least 80%, preferably at least 85%, more preferably at least 90%, more preferably at least 94% sequence identity therewith, and a reverse primer comprising a sequence of SEQ ID NO:63 or a sequence having at least 80%, preferably at least 85%, more preferably at least 90%, more preferably at least 94% sequence identity therewith, and
   h. A forward primer comprising a sequence of SEQ ID NO:65 or a sequence having at least 80%, preferably at least 85%, more preferably at least 90%, more preferably at least 94% sequence identity therewith, and a reverse primer comprising a sequence of SEQ ID NO:66 or a sequence having at least 80%, preferably at least 85%, more preferably at least 90%, more preferably at least 94% sequence identity therewith.

All primers of the primer pairs indicated under points a. to h. above may comprise a sequence having at least 80%, more preferably at least 85%, more preferably at least 90%, more preferably at least 94% sequence identity with the sequences of the indicated SEQ ID NOs.

### Preferably, the kit additionally comprises

(ii) the following extension primers:
   a. KPC-2 extension primer comprising a sequence of SEQ ID NO:3 or a sequence having a sequence identity of at least 80%, more preferably at least 85% more preferably at least 90%, more preferably at least 93% with SEQ ID NO:3,
   b. NDM-1 extension primer comprising a sequence of SEQ ID NO:50 or a sequence having a sequence identity of at least 80%, more preferably at least 85% more preferably at least 90%, more preferably at least 93% with SEQ ID NO:50,
   c. OXA-9 extension primer comprising a sequence of SEQ ID NO:53 or a sequence having a sequence identity of at least 80%, more preferably at least 85% more preferably at least 90%, more preferably at least 93% with SEQ ID NO:53,
   d. OXA-48 extension primer comprising a sequence of SEQ ID NO:56 or a sequence having a sequence identity of at least 80%, more preferably at least 85% more preferably at least 90%, more preferably at least 93% with SEQ ID NO:56,
   e. CTX-M-9 extension primer comprising a sequence of SEQ ID NO:59 or a sequence having a sequence identity of at least 80%, more preferably at least 85% more preferably at least 90%, more preferably at least 93% with SEQ ID NO:59,
   f. CTX-M-15 extension primer comprising a sequence of SEQ ID NO:18 or a sequence having a sequence identity of at least 80%, more preferably at least 85% more preferably at least 90%, more preferably at least 93% with SEQ ID NO:18,
   g. AAC(6')-lb-cr (223) extension primer comprising a sequence of SEQ ID NO:64 or a sequence having a sequence identity of at least 80%, more preferably at least 85% more preferably at least 90%, more preferably at least 93% with SEQ ID NO:64, and
   h. AAC(6')-lb-cr (454) extension primer comprising a sequence of SEQ ID NO:24 or a sequence having a sequence identity of at least 80%, more preferably at least 85% more preferably at least 90%, more preferably at least 93% with SEQ ID NO:24.

All primers indicated under points a. to h. above may comprise a sequence having at least 80%, more preferably at least 85%, more preferably at least 90%, more preferably at least 93% sequence identity with the sequences of the indicated SEQ ID NOs.

In embodiments of the present invention, the amplification primers do not only comprise the sequences indicated above or variants having a sequence identity of at least 80%, more preferably at least 85% more preferably at least 90%, more preferably at least 94% therewith but even consist of these sequences. However, the present inventors found that the amplification is advantageously balanced if the amplification primers additionally comprise nucleotides at the 5'-end. Furthermore, this increases the masses of unused primers (out of the mass range on the spectra).

In embodiments of the present invention, the extension primers do not only comprise the sequences indicated above or variants having a sequence identity of at least 80%, more preferably at least 85% more preferably at least 90%, more preferably at least 93% therewith but even consist of these sequences. However, the present inventors found that it may be advantageous if non-complementary bases are added at the 5'-end of the primers so that each primer has a detectable different mass.

A particularly preferred kit of the present invention is a kit for performing the methods of the invention, wherein the kit comprises
(ii) the following amplification primers:
   a. A forward primer comprising a sequence of SEQ ID NO:1 or a sequence having at least 80%, preferably at least 85%, more preferably at least 90%, more preferably at least 95% sequence identity therewith, and a reverse primer comprising a sequence of SEQ ID NO:2 or a sequence having at least 80%, preferably at least 85%, more preferably at least 90%, more preferably at least 95% sequence identity therewith,
   b. A forward primer comprising a sequence of SEQ ID NO:4 or a sequence having at least 80%, preferably at least 85%, more preferably at least 90%, more preferably at least 95% sequence identity therewith, and a reverse primer comprising a sequence of SEQ ID NO:5 or a sequence having at least 80%, preferably at least 85%, more preferably at least 90%, more preferably at least 95% sequence identity therewith,
   c. A forward primer comprising a sequence of SEQ ID NO:7 or a sequence having at least 80%, preferably at least 85%, more preferably at least 90%, more preferably at least 95% sequence identity therewith, and a reverse primer comprising a sequence of SEQ ID NO:8 or a sequence having at least 80%, preferably at least 85%, more preferably at least 90%, more preferably at least 95% sequence identity therewith,
   d. A forward primer comprising a sequence of SEQ ID NO:10 or a sequence having at least 80%, preferably at least 85%, more preferably at least 90%, more preferably at least 95% sequence identity therewith, and a reverse primer comprising a sequence of SEQ ID NO:11 or a sequence having at least 80%, preferably at least 85%, more preferably at least 90%, more preferably at least 95% sequence identity therewith,
   e. A forward primer comprising a sequence of SEQ ID NO:13 or a sequence having at least 80%, preferably at least 85%, more preferably at least 90%, more preferably at least 95% sequence identity therewith, and a reverse primer comprising a sequence of SEQ ID NO:14 or a sequence having at least 80%, preferably at least 85%, more preferably at least 90%, more preferably at least 95% sequence identity therewith,
   f. A forward primer comprising a sequence of SEQ ID NO:16 or a sequence having at least 80%, preferably at least 85%, more preferably at least 90%, more preferably at least 95% sequence identity therewith, and a reverse primer comprising a sequence of SEQ ID NO:17 or a sequence having at least 80%, preferably at least 85%, more preferably at least 90%, more preferably at least 95% sequence identity therewith,
   g. A forward primer comprising a sequence of SEQ ID NO:19 or a sequence having at least 80%, preferably at least 85%, more preferably at least 90%, more preferably at least 95% sequence identity therewith, and a reverse primer comprising a sequence of SEQ ID NO:20 or a sequence having at least 80%, preferably at least 85%, more preferably at least 90%, more preferably at least 95% sequence identity therewith, and
   h. A forward primer comprising a sequence of SEQ ID NO:22 or a sequence having at least 80%, preferably at least 85%, more preferably at least 90%, more preferably at least 95% sequence identity therewith, and a reverse primer comprising a sequence of SEQ ID NO:23 or a sequence having at least 80%, preferably at least 85%, more preferably at least 90%, more preferably at least 95% sequence identity therewith.

All primers of the primer pairs indicated under points a. to h. above may comprise a sequence having at least 80%, more preferably at least 85%, more preferably at least 90%, more preferably at least 95% sequence identity with the sequences of the indicated SEQ ID NOs.

### Preferably, the kit additionally comprises

(iii) the following extension primers:
   a. KPC-2 extension primer comprising a sequence of SEQ ID NO:3 or a sequence having a sequence identity of at least 80%, more preferably at least 85% more preferably at least 90%, more preferably at least 93% with SEQ ID NO:3,
   b. NDM-1 extension primer comprising a sequence of SEQ ID NO:6 or a sequence having a sequence identity of at least 80%, more preferably at least 85% more preferably at least 90%, more preferably at least 93% with SEQ ID NO:6,
   c. OXA-9 extension primer comprising a sequence of SEQ ID NO:9 or a sequence having a sequence identity of at least 80%, more preferably at least 85% more preferably at least 90%, more preferably at least 93% with SEQ ID NO:9,
   d. OXA-48 extension primer comprising a sequence of SEQ ID NO:12 or a sequence having a sequence identity of at least 80%, more preferably at least 85% more preferably at least 90%, more preferably at least 93% with SEQ ID NO:12,
   e. CTX-M-9 extension primer comprising a sequence of SEQ ID NO:15 or a sequence having a sequence identity of at least 80%, more preferably at least 85% more preferably at least 90%, more preferably at least 93% with SEQ ID NO:15,
   f. CTX-M-15 extension primer comprising a sequence of SEQ ID NO:18 or a sequence having a sequence identity of at least 80%, more preferably at least 85% more preferably at least 90%, more preferably at least 93% with SEQ ID NO:18,
   g. AAC(6')-lb-cr (223) extension primer comprising a sequence of SEQ ID NO:21 or a sequence having a sequence identity of at least 80%, more preferably at least 85% more preferably at least 90%, more preferably at least 93% with SEQ ID NO:21, and
   h. AAC(6')-lb-cr (454) extension primer comprising a sequence of SEQ ID NO:24 or a sequence having a sequence identity of at least 80%, more preferably at least 85% more preferably at least 90%, more preferably at least 93% with SEQ ID NO:24.

All primers indicated under points a. to h. above may comprise a sequence having at least 80%, more preferably at least 85%, more preferably at least 90%, more preferably at least 93% sequence identity with the sequences of the indicated SEQ ID NOs.

Preferably, the amplifying primers do not only comprise the sequences indicated above or variants having a sequence identity of at least 80%, more preferably at least 85% more preferably at least 90%, more preferably at least 95% therewith but even consist of these sequences.

Preferably, the extension primers indicated above do not only comprise the sequences indicated above or variants having a sequence identity of at least 80%, more preferably at least 85% more preferably at least 90%, more preferably at least 93% therewith but even consist of these sequences.

In preferred embodiments, the kits of the present invention comprise further primers. Particularly preferably, the kits of the invention additionally comprise
(i) at least one, more preferably at least two, more preferably at least three, more preferably at least four, more preferably at least five, more preferably all six of the following amplification primer pairs:
   i. Primer pair 9 consisting of a forward primer comprising a sequence of SEQ ID NO:67 or a sequence having at least 80%, preferably at least 85%, more preferably at least 90%, more preferably at least 94% sequence identity therewith, and a reverse primer comprising a sequence of SEQ ID NO:68 or a sequence having at least 80%, preferably at least 85%, more preferably at least 90%, more preferably at least 94% sequence identity therewith,
   j. Primer pair 10 consisting of a forward primer comprising a sequence of SEQ ID NO:73 or a sequence having at least 80%, preferably at least 85%, more preferably at least 90%, more preferably at least 94% sequence identity therewith, and a reverse primer comprising a sequence of SEQ ID NO:74 or a sequence having at least 80%, preferably at least 85%, more preferably at least 90%, more preferably at least 94% sequence identity therewith,
   k. Primer pair 11 consisting of a forward primer comprising a sequence of SEQ ID NO:76 or a sequence having at least 80%, preferably at least 85%, more preferably at least 90%, more preferably at least 94% sequence identity therewith, and a reverse primer comprising a sequence of SEQ ID NO:77 or a sequence having at least 80%, preferably at least 85%, more preferably at least 90%, more preferably at least 94% sequence identity therewith,
   l. Primer pair 12 consisting of a forward primer comprising a sequence of SEQ ID NO:79 or a sequence having at least 80%, preferably at least 85%, more preferably at least 90%, more preferably at least 94% sequence identity therewith, and a reverse primer comprising a sequence of SEQ ID NO:80 or a sequence having at least 80%, preferably at least 85%, more preferably at least 90%, more preferably at least 94% sequence identity therewith,
   m. Primer pair 13 consisting of a forward primer comprising a sequence of SEQ ID NO:82 or a sequence having at least 80%, preferably at least 85%, more preferably at least 90%, more preferably at least 94% sequence identity therewith, and a reverse primer comprising a sequence of SEQ ID NO:83 or a sequence having at least 80%, preferably at least 85%, more preferably at least 90%, more preferably at least 94% sequence identity therewith, and
   n. Primer pair 14 consisting of a forward primer comprising a sequence of SEQ ID NO:84 or a sequence having at least 80%, preferably at least 85%, more preferably at least 90%, more preferably at least 94% sequence identity therewith, and a reverse primer comprising a sequence of SEQ ID NO:85 or a sequence having at least 80%, preferably at least 85%, more preferably at least 90%, more preferably at least 94% sequence identity therewith.

All primers of the primer pairs indicated under points i. to n. above may comprise a sequence having at least 80%, more preferably at least 85%, more preferably at least 90%, more preferably at least 94% sequence identity with the sequences of the indicated SEQ ID NOs.

In embodiments of the present invention, the forward and reverse primers of primer pairs 9 to 14 do not only comprise the sequences of SEQ ID NOs: 67, 68, 73, 74, 76, 77, 79, 80, 82-85 as indicated above or variants having a sequence identity of at least 80%, more preferably at least 85% more preferably at least 90%, more preferably at least 94% therewith but even consist of these sequences. However, the present inventors found that the amplification is advantageously balanced if the forward and reverse primers additionally comprise nucleotides at the 5'-end. Furthermore, this increases the masses of unused primers (out of the mass range on the spectra).

Therefore, a particularly preferred kit of the present invention comprises
(i) at least one, more preferably at least two, more preferably at least three, more preferably at least four, more preferably at least five, more preferably all six of the following primer pairs:
   i. Primer pair 9 consisting of a forward primer comprising a sequence of SEQ ID NO:25 or a sequence having at least 80%, preferably at least 85%, more preferably at least 90%, more preferably at least 95% sequence identity therewith, and a reverse primer comprising a sequence of SEQ ID NO:26 or a sequence having at least 80%, preferably at least 85%, more preferably at least 90%, more preferably at least 95% sequence identity therewith,
   j. Primer pair 10 consisting of a forward primer comprising a sequence of SEQ ID NO:31 or a sequence having at least 80%, preferably at least 85%, more preferably at least 90%, more preferably at least 95% sequence identity therewith, and a reverse primer comprising a sequence of SEQ ID NO:32 or a sequence having at least 80%, preferably at least 85%, more preferably at least 90%, more preferably at least 95% sequence identity therewith,
   k. Primer pair 11 consisting of a forward primer comprising a sequence of SEQ ID NO:34 or a sequence having at least 80%, preferably at least 85%, more preferably at least 90%, more preferably at least 95% sequence identity therewith, and a reverse primer comprising a sequence of SEQ ID NO:35 or a sequence having at least 80%, preferably at least 85%, more preferably at least 90%, more preferably at least 95% sequence identity therewith,
   l. Primer pair 12 consisting of a forward primer comprising a sequence of SEQ ID NO:37 or a sequence having at least 80%, preferably at least 85%, more preferably at least 90%, more preferably at least 95% sequence identity therewith, and a reverse primer comprising a sequence of SEQ ID NO:38 or a sequence having at least 80%, preferably at least 85%, more preferably at least 90%, more preferably at least 95% sequence identity therewith,
   m. Primer pair 13 consisting of a forward primer comprising a sequence of SEQ ID NO:40 or a sequence having at least 80%, preferably at least 85%, more preferably at least 90%, more preferably at least 95% sequence identity therewith, and a reverse primer comprising a sequence of SEQ ID NO:41 or a sequence having at least 80%, preferably at least 85%, more preferably at least 90%, more preferably at least 95% sequence identity therewith, and
   n. Primer pair 14 consisting of a forward primer comprising a sequence of SEQ ID NO:43 or a sequence having at least 80%, preferably at least 85%, more preferably at least 90%, more preferably at least 95% sequence identity therewith, and a reverse primer comprising a sequence of SEQ ID NO:44 or a sequence having at least 80%, preferably at least 85%, more preferably at least 90%, more preferably at least 95% sequence identity therewith.

All primers of the primer pairs indicated under points i. to n. above may comprise a sequence having at least 80%, more preferably at least 85%, more preferably at least 90%, more preferably at least 95% sequence identity with the sequences of the indicated SEQ ID NOs.

In preferred embodiments of the present invention, the forward and reverse primers do not only comprise the sequences of SEQ ID NOs: 25, 26, 31, 32, 34, 35, 37, 38, 40, 41, 43, 44 as indicated above or variants having a sequence identity of at least 80%, more preferably at least 85% more preferably at least 90%, more preferably at least 95% therewith but even consist of these sequences.

Preferably, the kits of the invention further comprise extension primers corresponding to primer pairs 9 to 14, wherein both gapA (420) and gapA (456) extension primer correspond to primer pair 9. Preferably, the kits comprise
(ii) at least one, more preferably at least two, more preferably at least three, more preferably at least four, more preferably at least five, more preferably at least six and even more preferably all seven of the following extension primers:
   i. gapA (420) extension primer comprising a sequence of SEQ ID NO:69 or a sequence having a sequence identity of at least 80%, more preferably at least 85% more preferably at least 90%, more preferably at least 93% with SEQ ID NO:69,
   j. gapA (456) extension primer comprising a sequence of SEQ ID NO:72 or a sequence having a sequence identity of at least 80%, more preferably at least 85% more preferably at least 90%, more preferably at least 93% with SEQ ID NO:72,
   k. infB (1608) extension primer comprising a sequence of SEQ ID NO:75 or a sequence having a sequence identity of at least 80%, more preferably at least 85% more preferably at least 90%, more preferably at least 93% with SEQ ID NO:75,
   l. mdh (633) extension primer comprising a sequence of SEQ ID NO:78 or a sequence having a sequence identity of at least 80%, more preferably at least 85% more preferably at least 90%, more preferably at least 93% with SEQ ID NO:78,
   m. phoE (732) extension primer comprising a sequence of SEQ ID NO:81 or a sequence having a sequence identity of at least 80%, more preferably at least 85% more preferably at least 90%, more preferably at least 93% with SEQ ID NO:81,
   n. phoE (867) extension primer comprising a sequence of SEQ ID NO:42 or a sequence having a sequence identity of at least 80%, more preferably at least 85% more preferably at least 90%, more preferably at least 93% with SEQ ID NO:42,
   o. rpoB (1819) extension primer comprising a sequence of SEQ ID NO:86 or a sequence having a sequence identity of at least 80%, more preferably at least 85% more preferably at least 90%, more preferably at least 93% with SEQ ID NO:86.

All primers indicated under points i. to o. above may comprise a sequence having at least 80%, more preferably at least 85%, more preferably at least 90%, more preferably at least 93% sequence identity with the sequences of the indicated SEQ ID NOs.

In embodiments of the present invention, the extension primers do not only comprise the sequences indicated above or variants having a sequence identity of at least 80%, more preferably at least 85% more preferably at least 90%, more preferably at least 93% therewith but even consist of these sequences. However, the present inventors found that it may be advantageous if non-complementary bases are added at the 5'-end of the primers so that each primer has a detectable different mass.

A particularly preferred kit of the present invention comprises
(ii) at least one, more preferably at least two, more preferably at least three, more preferably at least four, more preferably at least five, more preferably at least six and even more preferably all seven of the following extension primers:
   i. gapA (420) extension primer comprising a sequence of SEQ ID NO:27 or a sequence having a sequence identity of at least 80%, more preferably at least 85% more preferably at least 90%, more preferably at least 93% with SEQ ID NO:27,
   j. gapA (456) extension primer comprising a sequence of SEQ ID NO:30 or a sequence having a sequence identity of at least 80%, more preferably at least 85% more preferably at least 90%, more preferably at least 93% with SEQ ID NO:30,
   k. infB (1608) extension primer comprising a sequence of SEQ ID NO:33 or a sequence having a sequence identity of at least 80%, more preferably at least 85% more preferably at least 90%, more preferably at least 93% with SEQ ID NO:33,
   l. mdh (633) extension primer comprising a sequence of SEQ ID NO:36 or a sequence having a sequence identity of at least 80%, more preferably at least 85% more preferably at least 90%, more preferably at least 93% with SEQ ID NO:36,
   m. phoE (732) extension primer comprising a sequence of SEQ ID NO:39 or a sequence having a sequence identity of at least 80%, more preferably at least 85% more preferably at least 90%, more preferably at least 93% with SEQ ID NO:39,
   n. phoE (867) extension primer comprising a sequence of SEQ ID NO:42 or a sequence having a sequence identity of at least 80%, more preferably at least 85% more preferably at least 90%, more preferably at least 93% with SEQ ID NO:42,
   o. rpoB (1819) extension primer comprising a sequence of SEQ ID NO:45 or a sequence having a sequence identity of at least 80%, more preferably at least 85% more preferably at least 90%, more preferably at least 93% with SEQ ID NO:45.

All primers indicated under points i. to o. above may comprise a sequence having at least 80%, more preferably at least 85%, more preferably at least 90%, more preferably at least 94% sequence identity with the sequences of the indicated SEQ ID NOs.

Preferably, the extension primers indicated above do not only comprise the sequences indicated above or variants having a sequence identity of at least 80%, more preferably at least 85% more preferably at least 90%, more preferably at least 93% therewith but even consist of these sequences.

A particularly preferred kit of the present invention comprises
(i) the following amplification primers:
   a. A forward primer comprising a sequence of SEQ ID NO:1 or a sequence having at least 80%, preferably at least 85%, more preferably at least 90%, more preferably at least 95% sequence identity therewith, and a reverse primer comprising a sequence of SEQ ID NO:2 or a sequence having at least 80%, preferably at least 85%, more preferably at least 90%, more preferably at least 95% sequence identity therewith,
   b. A forward primer comprising a sequence of SEQ ID NO:4 or a sequence having at least 80%, preferably at least 85%, more preferably at least 90%, more preferably at least 95% sequence identity therewith, and a reverse primer comprising a sequence of SEQ ID NO:5 or a sequence having at least 80%, preferably at least 85%, more preferably at least 90%, more preferably at least 95% sequence identity therewith,
   c. A forward primer comprising a sequence of SEQ ID NO:7 or a sequence having at least 80%, preferably at least 85%, more preferably at least 90%, more preferably at least 95% sequence identity therewith, and a reverse primer comprising a sequence of SEQ ID NO:8 or a sequence having at least 80%, preferably at least 85%, more preferably at least 90%, more preferably at least 95% sequence identity therewith,
   d. A forward primer comprising a sequence of SEQ ID NO:10 or a sequence having at least 80%, preferably at least 85%, more preferably at least 90%, more preferably at least 95% sequence identity therewith, and a reverse primer comprising a sequence of SEQ ID NO:11 or a sequence having at least 80%, preferably at least 85%, more preferably at least 90%, more preferably at least 95% sequence identity therewith,
   e. A forward primer comprising a sequence of SEQ ID NO:13 or a sequence having at least 80%, preferably at least 85%, more preferably at least 90%, more preferably at least 95% sequence identity therewith, and a reverse primer comprising a sequence of SEQ ID NO:14 or a sequence having at least 80%, preferably at least 85%, more preferably at least 90%, more preferably at least 95% sequence identity therewith,
   f. A forward primer comprising a sequence of SEQ ID NO:16 or a sequence having at least 80%, preferably at least 85%, more preferably at least 90%, more preferably at least 95% sequence identity therewith, and a reverse primer comprising a sequence of SEQ ID NO:17 or a sequence having at least 80%, preferably at least 85%, more preferably at least 90%, more preferably at least 95% sequence identity therewith,
   g. A forward primer comprising a sequence of SEQ ID NO:19 or a sequence having at least 80%, preferably at least 85%, more preferably at least 90%, more preferably at least 95% sequence identity therewith, and a reverse primer comprising a sequence of SEQ ID NO:20 or a sequence having at least 80%, preferably at least 85%, more preferably at least 90%, more preferably at least 95% sequence identity therewith,
   h. A forward primer comprising a sequence of SEQ ID NO:22 or a sequence having at least 80%, preferably at least 85%, more preferably at least 90%, more preferably at least 95% sequence identity therewith, and a reverse primer comprising a sequence of SEQ ID NO:23 or a sequence having at least 80%, preferably at least 85%, more preferably at least 90%, more preferably at least 95% sequence identity therewith,
   i. A forward primer comprising a sequence of SEQ ID NO:25 or a sequence having at least 80%, preferably at least 85%, more preferably at least 90%, more preferably at least 95% sequence identity therewith, and a reverse primer comprising a sequence of SEQ ID NO:26 or a sequence having at least 80%, preferably at least 85%, more preferably at least 90%, more preferably at least 95% sequence identity therewith,
   j. A forward primer comprising a sequence of SEQ ID NO:31 or a sequence having at least 80%, preferably at least 85%, more preferably at least 90%, more preferably at least 95% sequence identity therewith, and a reverse primer comprising a sequence of SEQ ID NO:32 or a sequence having at least 80%, preferably at least 85%, more preferably at least 90%, more preferably at least 95% sequence identity therewith,
   k. A forward primer comprising a sequence of SEQ ID NO:34 or a sequence having at least 80%, preferably at least 85%, more preferably at least 90%, more preferably at least 95% sequence identity therewith, and a reverse primer comprising a sequence of SEQ ID NO:35 or a sequence having at least 80%, preferably at least 85%, more preferably at least 90%, more preferably at least 95% sequence identity therewith,
   l. A forward primer comprising a sequence of SEQ ID NO:37 or a sequence having at least 80%, preferably at least 85%, more preferably at least 90%, more preferably at least 95% sequence identity therewith, and a reverse primer comprising a sequence of SEQ ID NO:38 or a sequence having at least 80%, preferably at least 85%, more preferably at least 90%, more preferably at least 95% sequence identity therewith,
   m. A forward primer comprising a sequence of SEQ ID NO:40 or a sequence having at least 80%, preferably at least 85%, more preferably at least 90%, more preferably at least 95% sequence identity therewith, and a reverse primer comprising a sequence of SEQ ID NO:41 or a sequence having at least 80%, preferably at least 85%, more preferably at least 90%, more preferably at least 95% sequence identity therewith, and
   n. A forward primer comprising a sequence of SEQ ID NO:43 or a sequence having at least 80%, preferably at least 85%, more preferably at least 90%, more preferably at least 95% sequence identity therewith, and a reverse primer comprising a sequence of SEQ ID NO:44 or a sequence having at least 80%, preferably at least 85%, more preferably at least 90%, more preferably at least 95% sequence identity therewith, and
(ii) the following extension primers:
   a. KPC-2 extension primer comprising a sequence of SEQ ID NO:3 or a sequence having a sequence identity of at least 80%, more preferably at least 85% more preferably at least 90%, more preferably at least 93% with SEQ ID NO:3,
   b. NDM-1 extension primer comprising a sequence of SEQ ID NO:6 or a sequence having a sequence identity of at least 80%, more preferably at least 85% more preferably at least 90%, more preferably at least 93% with SEQ ID NO:6,
   c. OXA-9 extension primer comprising a sequence of SEQ ID NO:9 or a sequence having a sequence identity of at least 80%, more preferably at least 85% more preferably at least 90%, more preferably at least 93% with SEQ ID NO:9,
   d. OXA-48 extension primer comprising a sequence of SEQ ID NO:12 or a sequence having a sequence identity of at least 80%, more preferably at least 85% more preferably at least 90%, more preferably at least 93% with SEQ ID NO:12,
   e. CTX-M-9 extension primer comprising a sequence of SEQ ID NO:15 or a sequence having a sequence identity of at least 80%, more preferably at least 85% more preferably at least 90%, more preferably at least 93% with SEQ ID NO:15,
   f. CTX-M-15 extension primer comprising a sequence of SEQ ID NO:18 or a sequence having a sequence identity of at least 80%, more preferably at least 85% more preferably at least 90%, more preferably at least 93% with SEQ ID NO:18,
   g. AAC(6')-lb-cr (223) extension primer comprising a sequence of SEQ ID NO:21 or a sequence having a sequence identity of at least 80%, more preferably at least 85% more preferably at least 90%, more preferably at least 93% with SEQ ID NO:21,
   h. AAC(6')-lb-cr (454) extension primer comprising a sequence of SEQ ID NO:24 or a sequence having a sequence identity of at least 80%, more preferably at least 85% more preferably at least 90%, more preferably at least 93% with SEQ ID NO:24,
   i. gapA (420) extension primer comprising a sequence of SEQ ID NO:27 or a sequence having a sequence identity of at least 80%, more preferably at least 85% more preferably at least 90%, more preferably at least 93% with SEQ ID NO:27,
   j. gapA (456) extension primer comprising a sequence of SEQ ID NO:30 or a sequence having a sequence identity of at least 80%, more preferably at least 85% more preferably at least 90%, more preferably at least 93% with SEQ ID NO:30,
   k. infB (1608) extension primer comprising a sequence of SEQ ID NO:33 or a sequence having a sequence identity of at least 80%, more preferably at least 85% more preferably at least 90%, more preferably at least 93% with SEQ ID NO:33,
   l. mdh (633) extension primer comprising a sequence of SEQ ID NO:36 or a sequence having a sequence identity of at least 80%, more preferably at least 85% more preferably at least 90%, more preferably at least 93% with SEQ ID NO:36,
   m. phoE (732) extension primer comprising a sequence of SEQ ID NO:39 or a sequence having a sequence identity of at least 80%, more preferably at least 85% more preferably at least 90%, more preferably at least 93% with SEQ ID NO:39,
   n. phoE (867) extension primer comprising a sequence of SEQ ID NO:42 or a sequence having a sequence identity of at least 80%, more preferably at least 85% more preferably at least 90%, more preferably at least 93% with SEQ ID NO:42, and
   o. rpoB (1819) extension primer comprising a sequence of SEQ ID NO:45 or a sequence having a sequence identity of at least 80%, more preferably at least 85% more preferably at least 90%, more preferably at least 93% with SEQ ID NO:45.

All primers of the primer pairs indicated under points a. to n. above may comprise a sequence having at least 80%, more preferably at least 85%, more preferably at least 90%, more preferably at least 95% sequence identity with the sequences of the indicated SEQ ID NOs and all extension primers indicated under points a. to o. above may comprise a sequence having at least 80%, more preferably at least 85%, more preferably at least 90%, more preferably at least 93% sequence identity with the sequences of the indicated SEQ ID NOs.

Preferably, the primers indicated above do not only comprise the sequences indicated above or variants having the indicated sequence identity therewith but even consist of these sequences.

The kits of the present invention may further comprise reagents for performing a PCR reaction. Preferably, the kit further comprises at least one reagent selected from the group consisting of buffer solution, deoxynucleotides (dNTPs) and DNA polymerase. More preferably, the kit comprises buffer solution, deoxynucleotides (dNTPs) and DNA polymerase. Preferably, the DNA polymerase is selected from the group consisting of Taq polymerase, Pwo polymerase and Pfu polymerase. Pfu polymerase is most preferred because it creates very little mutations during DNA amplification.

In a particularly preferred embodiment, the kit further comprises chain terminating nucleotides. Preferred chain terminating nucleotides of the present invention are acyclonucleotides (acyNTPs). Acyclonucleotides enable elongation of extension primers by exactly one nucleotide. In an alternative embodiment, the kit may comprise ddNTPs as chain terminating nucleotides instead of acyNTPs.

Preferably, the kit further comprises an archaeon DNA polymerase, more preferably Therminator DNA Polymerase.

The present invention also comprises the use of the above described methods for determining from a bacterial sample information on both the phylogenetic origin and the antibiotic resistances of bacterial strains.

The present invention also comprises a method of diagnosis of a disease based on the information on both the phylogenetic origin and/or antibiotic resistances of the bacterial strain determined by the methods of the present invention described above. The method of diagnosis of a disease preferably comprises the step of identifying the disease based on the determined information on both the phylogenetic origin and antibiotic resistances of the bacterial strain.

The present invention also comprises a method of treatment of a disease comprising the step of identifying a suitable drug for treatment of the disease based on the diagnosis of the disease by means of the method of the present invention and preferably the additional step of administering the identified drug to a patient in need thereof.

The present invention also comprises a method of infection management comprising the step of tracking the course of infection spreading based on the information on the phylogenetic origin and/or antibiotic resistances of the bacterial strain determined by the methods of the present invention described above. Preferably, the method comprises the additional step of improving hygienic standards based on the information obtained on the course of infection spreading.

### Examples

### Example 1

The 8 universal resistance markers of the present invention for gram-negative bacteria (position 814 of the KPC-2 coding sequence (SEQ ID NO:87), position 82 of the NDM-1 coding sequence (SEQ ID NO:88), position 104 of the OXA-9 coding sequence (SEQ ID NO:89), position 186 of the OXA-48 coding sequence (SEQ ID NO:90), position 556 of the CTX-M-9 coding sequence (SEQ ID NO:91), position 453 of the CTX-M-15 coding sequence (SEQ ID NO:92), position 223 of the AAC(6')-lb-cr coding sequence (SEQ ID NO:93) and position 454 of the AAC(6')-lb-cr coding sequence (SEQ ID NO:93)) have been tested for their ability to enable phylogenetic analysis. 93 sequenced *K. pneumoniae* isolates were investigated.

PCR reactions comprising the following primer pairs were performed:
a. Primer pair 1 consisting of a forward primer consisting of a sequence of SEQ ID NO:1 and a reverse primer consisting of a sequence of SEQ ID NO:2,
b. Primer pair 2 consisting of a forward primer consisting of a sequence of SEQ ID NO:4 and a reverse primer consisting of a sequence of SEQ ID NO:5,
c. Primer pair 3 consisting of a forward primer consisting of a sequence of SEQ ID NO:7 and a reverse primer consisting of a sequence of SEQ ID NO:8,
d. Primer pair 4 consisting of a forward primer consisting of a sequence of SEQ ID NO:10 and a reverse primer consisting of a sequence of SEQ ID NO:11,
e. Primer pair 5 consisting of a forward primer consisting of a sequence of SEQ ID NO:13 and a reverse primer consisting of a sequence of SEQ ID NO:14,
f. Primer pair 6 consisting of a forward primer consisting of a sequence of SEQ ID NO:16 and a reverse primer consisting of a sequence of SEQ ID NO:17,
g. Primer pair 7 consisting of a forward primer consisting of a sequence of SEQ ID NO:19 and a reverse primer consisting of a sequence of SEQ ID NO:20, and
h. Primer pair 8 consisting of a forward primer consisting of a sequence of SEQ ID NO:22 and a reverse primer consisting of a sequence of SEQ ID NO:23.

Extension primers were allowed to bind to the PCR products obtained from the PCR reactions. In particular,
a. KPC-2 extension primer consisting of a sequence of SEQ ID NO:3 was allowed to bind to the PCR product of primer pair 1,
b. NDM-1 extension primer consisting of a sequence of SEQ ID NO:6 was allowed to bind to the PCR product of primer pair 2,
c. OXA-9 extension primer consisting of a sequence of SEQ ID NO:9 was allowed to bind to the PCR product of primer pair 3,
d. OXA-48 extension primer consisting of a sequence of SEQ ID NO:12 was allowed to bind to the PCR product of primer pair 4,
e. CTX-M-9 extension primer consisting of a sequence of SEQ ID NO:15 was allowed to bind to the PCR product of primer pair 5,
f. CTX-M-15 extension primer consisting of a sequence of SEQ ID NO:18 was allowed to bind to the PCR product of primer pair 6,
g. AAC(6')-lb-cr (223) extension primer consisting of a sequence of SEQ ID NO:21 was allowed to bind to the PCR product of primer pair 7, and
h. AAC(6')-lb-cr (454) extension primer consisting of a sequence of SEQ ID NO:24 was allowed to bind to the PCR product of primer pair 8.

Extension primers were elongated at the 3'-end by exactly one nucleotide. The elongated extension primers were analyzed by mass spectrometry and the nucleotide that has been added to the extension primers was determined.

Information on both the phylogenetic origin and antibiotic resistances of the bacterial strains was determined based on a comparison of the pattern of the determined nucleotides with respective predetermined patterns of bacterial strains, which phylogenetic origin and antibiotic resistances were known.

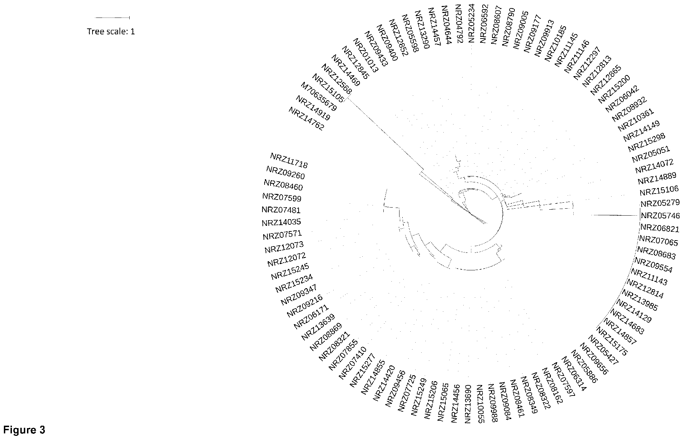

A phylogenetic tree was generated in MEGA6 using Neighbor Joining without bootstrapping. The resulting tree is shown in figure 3. The tree is based only on the resistance markers (SNP) including information about presence and absence of genes. The data show that information on the phylogenetic origin is derived from the analysis of the resistance markers of the present invention. Not every isolate was distinguishable but a satisfactory phylogenetic determination was achieved.

The analysis of the resistance markers of the present invention can be used to identify common and divergent patterns between different strains that were subjected to the analysis of the resistance markers. This information can be used to identify the phylogenetic relatedness of the strains. Many strains can be distinguished by the use of the resistance markers (SNP) including information about presence and absence of genes, however for some of the strains this information is not sufficient to differentiate them.

### Example 2

In another experiment, 7 phylogenetic *Klebsiella* markers (nucleotide at position 420 of the gapA coding sequence (SEQ ID NO:28), position 456 of the gapA coding sequence (SEQ ID NO:28), position 1608 of the infB coding sequence (SEQ ID NO:29), position 633 of the mdh coding sequence (SEQ ID NO:70), position 732 of the phoE coding sequence (SEQ ID NO:71), position 867 of the phoE coding sequence (SEQ ID NO:71) and position 1819 of the rpoB coding sequence (SEQ ID NO:94)) were tested for their discriminative power with regard to the 93 sequenced *K. pneumoniae* isolates described in example 1.

PCR reactions comprising the following primer pairs were performed:
i. Primer pair 9 consisting of a forward primer consisting of a sequence of SEQ ID NO:25 and a reverse primer consisting of a sequence of SEQ ID NO:26,
j. Primer pair 10 consisting of a forward primer consisting of a sequence of SEQ ID NO:31 and a reverse primer consisting of a sequence of SEQ ID NO:32,
k. Primer pair 11 consisting of a forward primer consisting of a sequence of SEQ ID NO:34 and a reverse primer consisting of a sequence of SEQ ID NO:35,
l. Primer pair 12 consisting of a forward primer consisting of a sequence of SEQ ID NO:37 and a reverse primer consisting of a sequence of SEQ ID NO:38,
m. Primer pair 13 consisting of a forward primer consisting of a sequence of SEQ ID NO:40 and a reverse primer consisting of a sequence of SEQ ID NO:41, and
n. Primer pair 14 consisting of a forward primer consisting of a sequence of SEQ ID NO:43 and a reverse primer consisting of a sequence of SEQ ID NO:44.

Extension primers were allowed to bind to the PCR products obtained from the PCR reactions. In particular,
i. gapA (420) extension primer consisting of a sequence of SEQ ID NO:27 was allowed to bind to the PCR product of primer pair 9,
j. gapA (456) extension primer consisting of a sequence of SEQ ID NO:30 was allowed to bind to the PCR product of primer pair 9,
k. infB (1608) extension primer consisting of a sequence of SEQ ID NO:33 was allowed to bind to the PCR product of primer pair 10,
l. mdh (633) extension primer consisting of a sequence of SEQ ID NO:36 was allowed to bind to the PCR product of primer pair 11,
m. phoE (732) extension primer consisting of a sequence of SEQ ID NO:39 was allowed to bind to the PCR product of primer pair 12,
n. phoE (867) extension primer consisting of a sequence of SEQ ID NO:42 was allowed to bind to the PCR product of primer pair 13, and
o. rpoB (1819) extension primer consisting of a sequence of SEQ ID NO:45 was allowed to bind to the PCR product of primer pair 14.

Extension primers were elongated at the 3'-end by exactly one nucleotide. The elongated extension primers were analyzed by mass spectrometry and the nucleotide that has been added to the extension primers was determined.

Information on the phylogenetic origin of the bacterial strains was determined based on a comparison of the pattern of the determined nucleotides with respective predetermined patterns of bacterial strains, which phylogenetic origin was known.

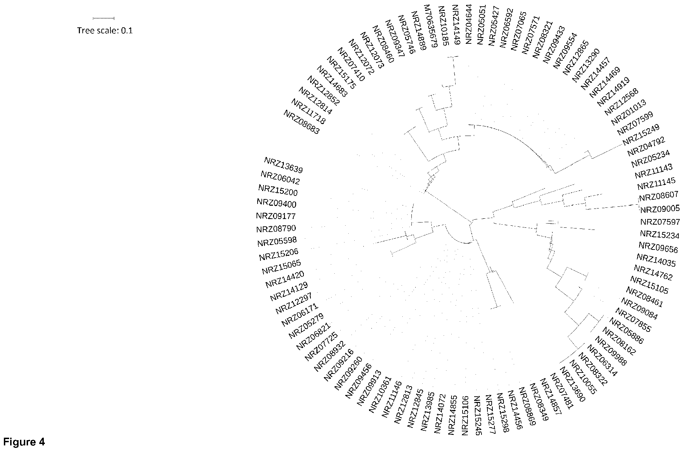

A phylogenetic tree was generated in MEGA6 using Neighbor Joining without bootstrapping. The resulting tree is shown in figure 4. The tree is based only on the phylogeny markers (SNP) including information about presence and absence of genes. As compared to example 1, testing the 7 phylogenetic markers results in an improved resolution of the phylogenetic analysis. However, also with the phylogenetic markers the results were not optimal. In particular, several distinct isolates were not distinguishable

### Example 3

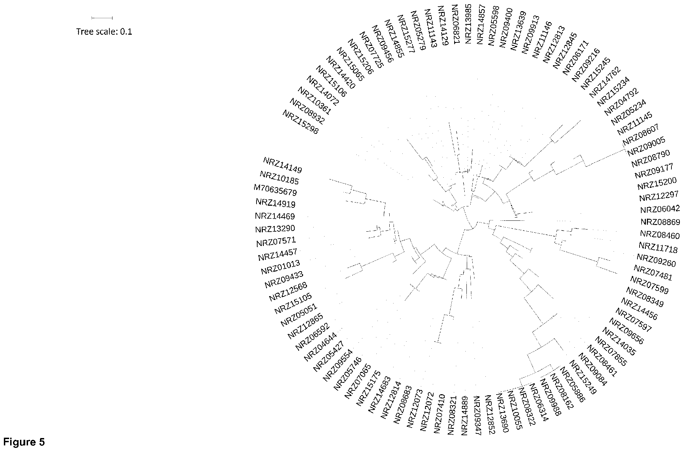

In a final experiment, a combined analysis utilizing information from both the 8 universal resistance markers and the 7 phylogenetic markers was performed. 93 sequenced *K. pneumoni*ae isolates were analyzed as described in examples 1 and 2. A phylogenetic tree was generated in MEGA6 using Neighbor Joining without bootstrapping. The resulting tree is shown in figure 5. The tree is based on both the resistance markers and the phylogeny markers including information about presence and absence of genes. The data show that combined testing of the 8 resistance markers and the 7 phylogenetic markers results in further improvement of the resolution of the phylogenetic analysis as compared to the results of example 2.

### Description of Figures

Figure 1 shows exemplarily binding regions of amplification primers and extension primers in the coding sequences of the antibiotic resistance genes. Binding regions of amplification primers are shown underlined. Binding regions of extension primers are shown in bold letters. Nucleotides of interest are in [brackets].
Figure 1A shows the KPC-2 coding sequence of SEQ ID NO:87 and exemplary primer binding regions and nucleotides of interest.
Figure 1B shows the NDM-1 coding sequence of SEQ ID NO:88 and exemplary primer binding regions and nucleotides of interest.
Figure 1C shows the OXA-9 coding sequence of SEQ ID NO:89 and exemplary primer binding regions and nucleotides of interest.
Figure 1D shows the OXA-48 coding sequence of SEQ ID NO:90 and exemplary primer binding regions and nucleotides of interest.
Figure 1E shows the CTX-M-9 coding sequence of SEQ ID NO:91 and exemplary primer binding regions and nucleotides of interest.
Figure 1F shows the CTX-M-15 coding sequence of SEQ ID NO:92 and exemplary primer binding regions and nucleotides of interest.
Figure 1G shows the AAC(6')-lb-cr coding sequence of SEQ ID NO:93 and exemplary primer binding regions and nucleotides of interest. Binding regions of two pairs of amplification primers and of two corresponding extension primers are shown.
Figure 1 shows exemplarily binding regions of amplification primers and extension primers in the coding sequences of the phylogenetic *Klebsiella* markers. Binding regions of amplification primers are shown underlined. Binding regions of extension primers are shown in bold letters. Nucleotides of interest are in [brackets].
Figure 2A shows the gapA coding sequence of SEQ ID NO:28 and exemplary primer binding regions and nucleotides of interest. Binding regions of two pairs of amplification primers and of two corresponding extension primers are shown.
Figure 2B shows residues 1 to 1852 of the infB coding sequence of SEQ ID NO:29 and exemplary primer binding regions and nucleotides of interest.
Figure 2C shows the mdh coding sequence of SEQ ID NO:70 and exemplary primer binding regions and nucleotides of interest.
Figure 2D shows the phoE coding sequence of SEQ ID NO:71 and exemplary primer binding regions and nucleotides of interest. Binding regions of two pairs of amplification primers and of two corresponding extension primers are shown. For better distinguishability the exemplary binding regions of one of the primer pairs are shown with increased font size. The double underlined residue is part of two exemplary primer binding regions.
Figure 2E shows residues 1465 to 2131 of the rpoB coding sequence of SEQ ID NO:94 and exemplary primer binding regions and nucleotides of interest.
Figure 3 shows a phylogenetic tree generated in MEGA6 using Neighbor Joining without bootstrapping. The scale bar shows the length of branch that represents an evolutionary distance of 1 nucleotide per position in the sequence. The tree is based only on the resistance markers (SNP) including information about presence and absence of genes. Identifiers of the 93 sequenced *K. pneumoniae* isolates are shown in the surrounding. The lines in the center indicate the phylogenetic grouping based on the results of experiment 1.
Figure 4 shows a phylogenetic tree generated in MEGA6 using Neighbor Joining without bootstrapping. The scale bar shows the length of branch that represents an evolutionary distance of 0.1 nucleotides per position in the sequence. The tree is based only on the on the phylogeny markers (SNP) including information about presence and absence of genes. Identifiers of the 93 sequenced *K. pneumoniae* isolates are shown in the surrounding. The lines in the center indicate the phylogenetic grouping based on the results of experiment 2.
Figure 5 shows a phylogenetic tree generated in MEGA6 using Neighbor Joining without bootstrapping. The scale bar shows the length of branch that represents an evolutionary distance of 0.1 nucleotides per position in the sequence. The tree is based on both the resistance markers and the phylogeny markers including information about presence and absence of genes. Identifiers of the 93 sequenced *K. pneumoniae* isolates are shown in the surrounding. The lines in the center indicate the phylogenetic grouping based on the results of experiment 3.

## Claims

1. Method for determining from a bacterial sample information on both the phylogenetic origin and the antibiotic resistances of bacterial strains, the method comprising the step of:
A) Performing PCR reactions comprising the following primer pairs:
a. Primer pair 1 consisting of a forward primer comprising a sequence of SEQ ID NO:46 or a sequence having at least 80% sequence identity therewith, and a reverse primer comprising a sequence of SEQ ID NO:47 or a sequence having at least 80% sequence identity therewith,
b. Primer pair 2 consisting of a forward primer comprising a sequence of SEQ ID NO:48 or a sequence having at least 80% sequence identity therewith, and a reverse primer comprising a sequence of SEQ ID NO:49 or a sequence having at least 80% sequence identity therewith,
c. Primer pair 3 consisting of a forward primer comprising a sequence of SEQ ID NO:51 or a sequence having at least 80% sequence identity therewith, and a reverse primer comprising a sequence of SEQ ID NO:52 or a sequence having at least 80% sequence identity therewith,
d. Primer pair 4 consisting of a forward primer comprising a sequence of SEQ ID NO:54 or a sequence having at least 80% sequence identity therewith, and a reverse primer comprising a sequence of SEQ ID NO:55 or a sequence having at least 80% sequence identity therewith,
e. Primer pair 5 consisting of a forward primer comprising a sequence of SEQ ID NO:57 or a sequence having at least 80% sequence identity therewith, and a reverse primer comprising a sequence of SEQ ID NO:58 or a sequence having at least 80% sequence identity therewith,
f. Primer pair 6 consisting of a forward primer comprising a sequence of SEQ ID NO:60 or a sequence having at least 80% sequence identity therewith, and a reverse primer comprising a sequence of SEQ ID NO:61 or a sequence having at least 80% sequence identity therewith,
g. Primer pair 7 consisting of a forward primer comprising a sequence of SEQ ID NO:62 or a sequence having at least 80% sequence identity therewith, and a reverse primer comprising a sequence of SEQ ID NO:63 or a sequence having at least 80% sequence identity therewith, and
h. Primer pair 8 consisting of a forward primer comprising a sequence of SEQ ID NO:65 or a sequence having at least 80% sequence identity therewith, and a reverse primer comprising a sequence of SEQ ID NO:66 or a sequence having at least 80% sequence identity therewith.

2. Method according to claim 1 comprising the step of:
A) Performing PCR reactions comprising the following primer pairs:
a. Primer pair 1 consisting of a forward primer comprising a sequence of SEQ ID NO:1 or a sequence having at least 80% sequence identity therewith, and a reverse primer comprising a sequence of SEQ ID NO:2 or a sequence having at least 80% sequence identity therewith,
b. Primer pair 2 consisting of a forward primer comprising a sequence of SEQ ID NO:4 or a sequence having at least 80% sequence identity therewith, and a reverse primer comprising a sequence of SEQ ID NO:5 or a sequence having at least 80% sequence identity therewith,
c. Primer pair 3 consisting of a forward primer comprising a sequence of SEQ ID NO:7 or a sequence having at least 80% sequence identity therewith, and a reverse primer comprising a sequence of SEQ ID NO:8 or a sequence having at least 80% sequence identity therewith,
d. Primer pair 4 consisting of a forward primer comprising a sequence of SEQ ID NO:10 or a sequence having at least 80% sequence identity therewith, and a reverse primer comprising a sequence of SEQ ID NO:11 or a sequence having at least 80% sequence identity therewith,
e. Primer pair 5 consisting of a forward primer comprising a sequence of SEQ ID NO:13 or a sequence having at least 80% sequence identity therewith, and a reverse primer comprising a sequence of SEQ ID NO:14 or a sequence having at least 80% sequence identity therewith,
f. Primer pair 6 consisting of a forward primer comprising a sequence of SEQ ID NO:16 or a sequence having at least 80% sequence identity therewith, and a reverse primer comprising a sequence of SEQ ID NO:17 or a sequence having at least 80% sequence identity therewith,
g. Primer pair 7 consisting of a forward primer comprising a sequence of SEQ ID NO:19 or a sequence having at least 80% sequence identity therewith, and a reverse primer comprising a sequence of SEQ ID NO:20 or a sequence having at least 80% sequence identity therewith, and
h. Primer pair 8 consisting of a forward primer comprising a sequence of SEQ ID NO:22 or a sequence having at least 80% sequence identity therewith, and a reverse primer comprising a sequence of SEQ ID NO:23 or a sequence having at least 80% sequence identity therewith.

3. Method according to claim 1 or 2 comprising the additional step of:
B) Allowing extension primers to bind to the PCR products obtained from the PCR reactions of step A).

4. Method according to claim 3, wherein at least one extension primer is binding to each of the PCR products obtained from step A).

5. Method according to claim 3 or 4, wherein the following extension primers are allowed to bind to the PCR products obtained from the PCR reactions of step A):
a. KPC-2 extension primer comprising a sequence of SEQ ID NO:3 or a sequence having a sequence identity of at least 80% with SEQ ID NO:3,
b. NDM-1 extension primer comprising a sequence of SEQ ID NO:50 or a sequence having a sequence identity of at least 80% with SEQ ID NO:50,
c. OXA-9 extension primer comprising a sequence of SEQ ID NO:53 or a sequence having a sequence identity of at least 80% with SEQ ID NO:53,
d. OXA-48 extension primer comprising a sequence of SEQ ID NO:56 or a sequence having a sequence identity of at least 80% with SEQ ID NO:56,
e. CTX-M-9 extension primer comprising a sequence of SEQ ID NO:59 or a sequence having a sequence identity of at least 80% with SEQ ID NO:59,
f. CTX-M-15 extension primer comprising a sequence of SEQ ID NO:18 or a sequence having a sequence identity of at least 80% with SEQ ID NO:18,
g. AAC(6')-lb-cr (223) extension primer comprising a sequence of SEQ ID NO:64 or a sequence having a sequence identity of at least 80% with SEQ ID NO:64, and
h. AAC(6')-lb-cr (454) extension primer comprising a sequence of SEQ ID NO:24 or a sequence having a sequence identity of at least 80% with SEQ ID NO:24.

6. Method according to at least one of claims 3 to 5, wherein following extension primers are allowed to bind to the PCR products obtained from the PCR reactions of step A):
a. KPC-2 extension primer comprising a sequence of SEQ ID NO:3 or a sequence having a sequence identity of at least 80% with SEQ ID NO:3,
b. NDM-1 extension primer comprising a sequence of SEQ ID NO:6 or a sequence having a sequence identity of at least 80% with SEQ ID NO:6,
c. OXA-9 extension primer comprising a sequence of SEQ ID NO:9 or a sequence having a sequence identity of at least 80% with SEQ ID NO:9,
d. OXA-48 extension primer comprising a sequence of SEQ ID NO:12 or a sequence having a sequence identity of at least 80% with SEQ ID NO:12,
e. CTX-M-9 extension primer comprising a sequence of SEQ ID NO:15 or a sequence having a sequence identity of at least 80% with SEQ ID NO:15,
f. CTX-M-15 extension primer comprising a sequence of SEQ ID NO:18 or a sequence having a sequence identity of at least 80% with SEQ ID NO:18,
g. AAC(6')-lb-cr (223) extension primer comprising a sequence of SEQ ID NO:21 or a sequence having a sequence identity of at least 80% with SEQ ID NO:21, and
h. AAC(6')-lb-cr (454) extension primer comprising a sequence of SEQ ID NO:24 or a sequence having a sequence identity of at least 80% with SEQ ID NO:24.

7. Method according to at least one of claims 1 to 6, wherein the PCR reactions of step A) additionally comprise at least one of the following primer pairs:
i. Primer pair 9 consisting of a forward primer comprising a sequence of SEQ ID NO:67 or a sequence having at least 80 sequence identity therewith, and a reverse primer comprising a sequence of SEQ ID NO:68 or a sequence having at least 80% sequence identity therewith,
j. Primer pair 10 consisting of a forward primer comprising a sequence of SEQ ID NO:73 or a sequence having at least 80% sequence identity therewith, and a reverse primer comprising a sequence of SEQ ID NO:74 or a sequence having at least 80% sequence identity therewith,
k. Primer pair 11 consisting of a forward primer comprising a sequence of SEQ ID NO:76 or a sequence having at least 80% sequence identity therewith, and a reverse primer comprising a sequence of SEQ ID NO:77 or a sequence having at least 80% sequence identity therewith,
l. Primer pair 12 consisting of a forward primer comprising a sequence of SEQ ID NO:79 or a sequence having at least 80% sequence identity therewith, and a reverse primer comprising a sequence of SEQ ID NO:80 or a sequence having at least 80% sequence identity therewith,
m. Primer pair 13 consisting of a forward primer comprising a sequence of SEQ ID NO:82 or a sequence having at least 80% sequence identity therewith, and a reverse primer comprising a sequence of SEQ ID NO:83 or a sequence having at least 80% sequence identity therewith, and
n. Primer pair 14 consisting of a forward primer comprising a sequence of SEQ ID NO:84 or a sequence having at least 80% sequence identity therewith, and a reverse primer comprising a sequence of SEQ ID NO:85 or a sequence having at least 80% sequence identity therewith.

8. Method according to claim 7, wherein additionally at least one of the following extension primers is allowed to bind to the PCR products obtained from the PCR reactions of step A):
i. gapA (420) extension primer comprising a sequence of SEQ ID NO:69 or a sequence having a sequence identity of at least 80% with SEQ ID NO:69,
j. gapA (456) extension primer comprising a sequence of SEQ ID NO:72 or a sequence having a sequence identity of at least 80% with SEQ ID NO:72,
k. infB (1608) extension primer comprising a sequence of SEQ ID NO:75 or a sequence having a sequence identity of at least 80% with SEQ ID NO:75,
l. mdh (633) extension primer comprising a sequence of SEQ ID NO:78 or a sequence having a sequence identity of at least 80% with SEQ ID NO:78,
m. phoE (732) extension primer comprising a sequence of SEQ ID NO:81 or a sequence having a sequence identity of at least 80% with SEQ ID NO:81,
n. phoE (867) extension primer comprising a sequence of SEQ ID NO:42 or a sequence having a sequence identity of at least 80% with SEQ ID NO:42, and
o. rpoB (1819) extension primer comprising a sequence of SEQ ID NO:86 or a sequence having a sequence identity of at least 80% with SEQ ID NO:86.

9. Method according to at least one of the preceding claims, wherein the bacterial strain is a multi-drug resistant gram-negative bacterium.

10. Method according to at least one of the preceding claims comprising the additional step of:
C) Elongating the extension primers at the 3'-end by at least one nucleotide.

11. Method according to claim 11 comprising the additional step of:
D) Identifying the at least one nucleotide that has been added to the extension primer.

12. Method according to at least one of claims 1 to 11 comprising the additional step of:
E) Determining information on both the phylogenetic origin and antibiotic resistances of the bacterial strain based on a comparison of the pattern of the determined nucleotides with respective predetermined patterns of bacterial strains, which phylogenetic origin and antibiotic resistances are known.

13. Kit comprising the following amplification primers:
a. A forward primer comprising a sequence of SEQ ID NO:46 or a sequence having at least 80% sequence identity therewith, and a reverse primer comprising a sequence of SEQ ID NO:47 or a sequence having at least 80% sequence identity therewith,
b. A forward primer comprising a sequence of SEQ ID NO:48 or a sequence having at least 80% sequence identity therewith, and a reverse primer comprising a sequence of SEQ ID NO:49 or a sequence having at least 80% sequence identity therewith,
c. A forward primer comprising a sequence of SEQ ID NO:51 or a sequence having at least 80% sequence identity therewith, and a reverse primer comprising a sequence of SEQ ID NO:52 or a sequence having at least 80% sequence identity therewith,
d. A forward primer comprising a sequence of SEQ ID NO:54 or a sequence having at least 80% sequence identity therewith, and a reverse primer comprising a sequence of SEQ ID NO:55 or a sequence having at least 80% sequence identity therewith,
e. A forward primer comprising a sequence of SEQ ID NO:57 or a sequence having at least 80% sequence identity therewith, and a reverse primer comprising a sequence of SEQ ID NO:58 or a sequence having at least 80% sequence identity therewith,
f. A forward primer comprising a sequence of SEQ ID NO:60 or a sequence having at least 80% sequence identity therewith, and a reverse primer comprising a sequence of SEQ ID NO:61 or a sequence having at least 80% sequence identity therewith,
g. A forward primer comprising a sequence of SEQ ID NO:62 or a sequence having at least 80% sequence identity therewith, and a reverse primer comprising a sequence of SEQ ID NO:63 or a sequence having at least 80% sequence identity therewith, and
h. A forward primer comprising a sequence of SEQ ID NO:65 or a sequence having at least 80% sequence identity therewith, and a reverse primer comprising a sequence of SEQ ID NO:66 or a sequence having at least 80% sequence identity therewith.

14. Kit according to claim 13 further comprising the following extension primers:
a. KPC-2 extension primer comprising a sequence of SEQ ID NO:3 or a sequence having a sequence identity of at least 80% with SEQ ID NO:3,
b. NDM-1 extension primer comprising a sequence of SEQ ID NO:50 or a sequence having a sequence identity of at least 80% with SEQ ID NO:50,
c. OXA-9 extension primer comprising a sequence of SEQ ID NO:53 or a sequence having a sequence identity of at least 80% with SEQ ID NO:53,
d. OXA-48 extension primer comprising a sequence of SEQ ID NO:56 or a sequence having a sequence identity of at least 80% with SEQ ID NO:56,
e. CTX-M-9 extension primer comprising a sequence of SEQ ID NO:59 or a sequence having a sequence identity of at least 80% with SEQ ID NO:59,
f. CTX-M-15 extension primer comprising a sequence of SEQ ID NO:18 or a sequence having a sequence identity of at least 80% with SEQ ID NO: 18,
g. AAC(6')-lb-cr (223) extension primer comprising a sequence of SEQ ID NO:64 or a sequence having a sequence identity of at least 80% with SEQ ID NO:64, and
h. AAC(6')-lb-cr (454) extension primer comprising a sequence of SEQ ID NO:24 or a sequence having a sequence identity of at least 80% with SEQ ID NO:24.

15. Use of a method for determining from a bacterial sample information on both the phylogenetic origin and the antibiotic resistances of bacterial strains, the method comprising the steps of:
A) determining which nucleotide is present
a. at position 814 in the KPC-2 coding sequence (SEQ ID NO:87),
b. at position 82 in the NDM-1 coding sequence (SEQ ID NO:88),
c. at position 104 in the OXA-9 coding sequence (SEQ ID NO:89),
d. at position 186 in the OXA-48 coding sequence (SEQ ID NO:90),
e. at position 556 in the CTX-M-9 coding sequence (SEQ ID NO:91),
f. at position 453 in the CTX-M-15 coding sequence (SEQ ID NO:92),
g. at position 223 in the AAC(6')-lb-cr coding sequence (SEQ ID NO:93), and
h. at position 454 in the AAC(6')-lb-cr coding sequence (SEQ ID NO:93),
or at analogous positions in variants or homologous coding sequences having at least 80% sequence identity with the indicated coding sequences, and
B) determining information on both the phylogenetic origin and antibiotic resistances of the bacterial strain based on a comparison of the pattern of the determined nucleotides with respective predetermined patterns of bacterial strains, which phylogenetic origin and antibiotic resistances are known.
